# EUROPEAN PATENT APPLICATION

(11) **EP 2 158 888 A1**
(43) Date of publication of application: **03.03.2010**
(21) Application number: 08163439.6
(22) Date of filing: 01.09.2008
(51) Int. Cl.: A61F 13/56, B32B 7/02, B32B 25/10, B32B 27/12

(54) **Activatable precursor of a composite laminate web and elastic composite laminate web**

(71) Applicant: 3M Innovative Properties Company, St. Paul, MN 55133-1000 (US)
(72) Inventor: Bosler, Manfred, 47877 Willich (DE); Jaeger, Tilman, 41564 Kaarst (DE)
(74) Representative: Wilhelm, Stefan

(57) **Abstract**

The present invention provides an activatable precursor of an elastic composite laminate web having a machine direction and a cross direction comprising an elastic film, a first non-woven web and a first layer of adhesive therebetween, said layer of adhesive comprising in CD two edge regions and an intermediate region between the two edge regions extending in MD, said layer of adhesive exhibiting an adhesive pattern so that the mean adhesive surface coverage measured in the cross direction comprises at least one maximum in each of the edge regions and at least one maximum proximate the center of the intermediate region and/or the center line of the web, respectively.

## Description

### Field of the invention

The present invention is directed to an activatable precursor of an elastic composite laminate comprising an elastic film and a non-woven web and to an elastic composite laminate web which is obtainable by stretching such precursor. The invention furthermore refers to a method of manufacturing such precursor web and such activated web, respectively.

### Background of the invention

Several stretch-activatable precursors of composite laminate webs and the corresponding elastic composite laminate webs obtainable therefrom by stretching, respectively, are known in the prior art. EP 1 900 512 Al discloses, for example, an activatable precursor of a zero strain composite laminate web comprising an activatable elastic laminate web having an elastic core layer and at least one skin layer, which is less elastic than the core layer. The precursor of such composite laminate web further comprises at least one pre-bonded staple fiber non-woven web which is attached to one of the skin layers of the elastic laminate web. The at least one staple fiber non-woven web has an elongation at break of at least 100 % in the cross direction and the activatable elastic laminate web forms an essentially homogenous microtextured surface when stretched in the first upload in the cross-direction past the elastic limit of the one or more skin layers.

WO 2006/124337 Al discloses a stretch laminate having a first substrate which is adhesively attached to an elastic film. The adhesive is applied in a continuous manner to the substrate in a first tack down region which is disposed proximate to an end of the stretch laminate. The adhesive is applied as a plurality of adhesive stripes in an activation region which is interior of the first tack down region. The adhesive stripes have a width and a distance between adjacent adhesive stripes. The ratio of stripe width to distance between the adhesive stripes is in the range of less than about 1 or greater than about 0.33.

WO 2006/063232 Al describes a stretch laminate having a first non-woven material, a second non-woven material and an elastic film. The elastic film may be interposed between said first and second non-woven material. The elastic film has a first longitudinal side edge and a second longitudinal side edge. The stretch laminate further has a first plane of adhesive having differing amounts of adhesive as measured laterally within the stretch laminate. The first plane of adhesive is interposed between the first non-woven material and the elastic film. The stretch laminate further has a second plane of adhesive having differing amounts of adhesive as measured laterally within the stretch laminate. The differing amounts of adhesive in said first and second plabe of adhesive, respectively, are controllable. The second plane of adhesive is interposed between the second non-woven material and the elastic film. Each of the above elements are essentially laminated together to form a composite laminate which forms the precursor of the stretch laminate. The laminate can be activated to form the stretch laminate.

US 2008/0038507 Al discloses an elastic laminate of a base layer with one or more attached elastic elements forming an elastic region. The elastic region width varies from a terminal end of the elastic tab to a proximal end of the elastic tab such that the width adjacent the terminal end is 20 to 80 % narrower than a width adjacent the proximal end. The elastic region is defined by a plurality of segments, wherein said segments have differing average widths in the length direction of the elastic tab region. The one or more elastic elements vary in one or more properties such that a plurality of the segments having different widths have substantially the same degree of elongation at a given elongation of the shaped elastic tab laminate.

WO 2008/036706 Al describes a method of making a plurality of diaper side panels from a continuous strip. The strip comprises a central region, a first lateral region, a second lateral region, a first set of fasteners anchored to the first lateral region and a second set of fasteners anchored to the second lateral region. The strip is separated in the machine direction to form a first set of the side panels and a second set of the side panels so that the side panels each include a fastener from the first set and the side panels each include a fastener from the second set.

US 2007/0254547 Al discloses a method for imparting elasticity to a precursor laminate comprising at least one elastic film having a width and at least one ply of non-woven fabric secured to the film, said method comprising the following steps: the laminate is unrolled in the form of a web in order to pass it between two sets of toothing, the teeth of which engage in each other in a direction perpendicular to the plane of the laminate in order to thus stretch the precursor laminate in its width, wherein a tension is imparted to the web in the longitudinal direction or in the machine direction during its unrolling between the toothing, particularly by providing a tensional roll downstream of the sets of toothing. US 2007/0254547 Al further describes a device for imparting elasticity to such precursor laminate as well as to a precursor laminate comprising at least one ply of non-woven material and at least one elastic film.

US 6 255 236 B1 discloses a laminate comprising an elastic web having a first surface and a second surface, a first non-woven bonded to the first surface of the elastic web and a second non-woven bonded to the second surface of the elastic web. Therein, a first region of said laminate defines an elastic lane and a second region of said laminate defines a first stiffened lane. Furthermore, a ratio of the force required to obtain 5 % deformation of said stiffened lane to the force required to obtain 5 % deformation of said elastic lane is at least approximately four.

As can be appreciated from the above-mentioned documents there are many different, sometimes conflicting demands with respect to the properties and characteristics of such elastic composite laminate webs. Since these products are typically mass-produced there is a need to improve the process of manufacturing by providing those properties and characteristics in a simple and cost-efficient manner. There is further a need for a way of optimizing and tailor-making the properties such as the elastic characteristics, the feeling to the skin and/or other tactile properties and/or the aesthetical appearance of activated composite laminate webs.

It is an object of the present invention to provide an activatable precursor of a composite laminate web which can be activated without damaging any layer of the composite laminate web. It is another object of the present invention to provide a method of manufacturing the activatable precursor of said elastic composite laminate web and the elastic composite laminate web obtainable therefrom by stretching, respectively, which is reliable and cost-efficient. It is another object of the present invention to provide an elastic composite laminate web with well-defined and/or adjustable elastic properties which is obtainable from said precursor web. These objects are achieved by the invention as defined by the claims.

### Summary of the invention

The present invention relates to an activatable precursor of an elastic composite laminate web having a machine direction and a cross direction comprising an elastic film, a first non-woven web and a first layer of adhesive therebetween, said layer of adhesive comprising in CD two edge regions and an intermediate region between the two edge regions extending in MD, said layer of adhesive exhibiting an adhesive pattern so that the mean adhesive surface coverage measured in the cross direction comprises at least one maximum in each of the edge regions and at least one maximum proximate the center of the intermediate region and/or the center line of the web, respectively. The present invention also relates to an activated composite laminate web obtainable from said precursor web by stretching it in CD. The present invention also refers to a method of manufacturing said precursor web and said activated web, respectively.

### Detailed description of the invention

The precursor of the composite laminate web is referred to above and below as "stretch-activatable" or "activatable". This means that the elasticity of the precursor can be increased by stretching it. If the precursor comprises a non-activated, i.e. not yet stretched elastic laminate comprising an elastic core layer and at least one less elastic skin layer stretching of the precursor affects both the elastic layer and the one or more non-woven web. The less elastic skin layer forms upon stretching beyond its elastic limit preferably a microstructured surface structure as is described in more detail below that renders the composite laminate web obtained by stretching, more elastic in subsequent stretching cycles, i.e. after the initial stretching cycle. Stretching of the precursor in the first stretching cycle furthermore provides an inelastic deformation of the non-woven web resulting in a higher elasticity of the composite laminate obtained by stretching, in subsequent stretching cycles relative to the elasticity displayed by the precursor in the first virgin stretching cycle. If the precursor comprises an activated, i.e. already stretched elastic laminate comprising an elastic core layer and at least one less elastic skin layer, or a one-layer elastic film made of an elastomeric material the first virgin stretching cycle applied to the precursor mainly results in an inelastic deformation of the one or two non-woven web layers. An elastomeric one-layer elastic film does not require activation, i.e. its elasticity is essentially not affected by the initial virgin stretching cycle.

The composite laminate web which is obtainable from the precursor by stretching is referred to above and below as "activated" or "stretch-activated". This means that elasticity of the stretched composite web is higher than the elasticity of the corresponding precursor prior to the initial virgin stretching cycle.

The present invention is based on the idea to vary and tailor-make the properties of an activated composite laminate web by stretch-activating the corresponding precursor laminate comprising at least one non-woven web and an elastic film which are bonded by an adhesive layer applied in a predetermined pattern or structure. The adhesive layer of the precursor comprises in cross direction (i.e. the direction normal to the direction of the running web, abbreviated as CD) two edge regions and an intermediate region therebetween both extending in machine direction (i.e. the direction of the running web, abbreviated as MD). The intermediate region furthermore comprises a center region which is arranged proximate the center line of the precursor web and the activated web, respectively, and/or to the center of the intermediate region, respectively. The center line extends in MD at the middle of the extension of the precursor web and the activated web, respectively, in CD. The center region extends around the center line in CD and/or is arranged proximate the center of the intermediate region of the precursor web or the activated web, respectively, in CD. The layer of adhesive exhibits an adhesive pattern so that the mean adhesive surface coverage measured in the cross direction comprises at least one maximum in each of the edge regions and at least one maximum in the center region.

The edge regions correspond to the areas where the precursor web is preferably gripped by the clamps, jaws or other gripping devices of a stretching apparatus when stretching the precursor web in CD thereby imparting an inhomogenous profile of elastic properties to the web in CD. Stretching can be performed, for example, in a tenter stretching apparatus or in a diverging disks stretching apparatus. The edge regions are preferably fully and continuously covered with adhesive and extend continuously in the machine direction along the length of the web at opposite sides. The edge regions preferably extend between from about 10 to about 30 mm and more preferably from about 15 to about 25 mm from the outer edge of the precursor web or activated web, in CD towards the center line. The intermediate region is arranged between the two edge regions.

Stretching of the precursor web or parts of the precursor web in CD, respectively, can also be effected by other stretching techniques including, for example, the so-called ring-rolling technique disclosed, e.g., in US 5,143,679.

Preferably, the width of the left and right edge regions, respectively, are essentially identical.

The mean adhesive surface coverage (as defined below) as measured in the cross direction comprises at least three maxima wherein each edge region exhibits at least one maximum and wherein the other maximum is located in the center region. The maxima are usually local maxima and may have an extension in CD of, for example, between 20 - 60 mm and more preferably of between 25 - 50 mm. The requirement that the maximum in the center region is "proximate to the center of the intermediate region and/or proximate to the center line of the web, respectively" means that at least part of such maximum preferably is within ± 25 mm, more preferably within ± 15 mm, even more preferably within ± 10 mm and especially preferably within ± 5 mm of said center of the intermediate region and/or said center line of the web, respectively. The requirement that each of the edge regions exhibits a maximum means that at least part of the extension of the maxima in the edge regions overlap with such edge regions.

In a preferred embodiment the intermediate region comprises n = 1, 3, 5, ..... maxima (with n being an integer) which are preferably arranged so that they are at essentially equal distances to each other. Embodiments wherein the intermediate region has 1, 3, 5 or 7 maxima and, in particular, 1, 3 or 5 maxima, are preferred. Thus, in the case of 1 maximum in the intermediate region, the precursor web comprises three maxima in total being arranged in each of the edge regions comprises and in the center region, respectively, which is arranged proximate the center line and/or the center of the intermediate region, respectively. In case of 3 maxima in the intermediate region, the precursor web comprises five maxima in total two of them being arranged in the edge regions. One of the 3 maxima located in the intermediate region is arranged in the center region proximate the center of the intermediate region and/or the center line, respectively, and the other two maxima are arranged in each case essentially at the center of the distance between the adjacent edge region and the center region, respectively. Precursor webs or activated webs, respectively exhibiting 3, 5 or 7 maxima of the mean adhesive surface coverage in CD are preferred.

Activated composite laminate webs having 3 maxima of the mean adhesive surface coverage can advantageously be processed in a waste-free manner to give a sequence of right and left ear panels which can be attached, for example, to the chassis of disposable products such as diapers. Likewise, activated composite laminate webs having 5 maxima of the mean adhesive surface coverage can advantageously be processed in a waste-free manner to give a twofold sequence of right and left ear panels, and so on.

The center region preferably has a width in CD between 20 and 60 mm and more preferably between 30 and 50 mm. The center region is preferably arranged symmetrically around the center line. In a preferred embodiment the center region is essentially fully coated with adhesive so that the center region forms a center adhesive stripe.

The adhesive layer or layers, respectively, can be arranged in various patterns but preferably comprises stripes of adhesive and stripes therebetween which exhibit less adhesive or are preferably essentially free of adhesive, respectively. The stripes are oriented and extend in the machine direction. The stripes are preferably defined by straight edge lines so that the stripes have an essentially rectangular shape extending in MD but other geometries such as, for example, wavy shapes are also possible. The stripes forming the edge region may exhibit, for example, straight edge lines or an outer straight edge line and an inner wavy edge line, respectively. The terms "outer" and "inner" edge line refer to the respective position relative to the center line of the precursor web or the activated web, respectively. The stripes of adhesive are preferably fully and continuously covered with adhesive but it is also possible that the stripes of adhesive exhibits segments of adhesives which are separated by adhesive-free areas or exhibits a sequence of individual adhesive elements such as adhesive dots, for example. The stripes between the adhesive stripes are preferably essentially free of adhesive.

The requirement of at least one maximum of the mean adhesive surface coverage of the adhesive layer in each of the edge region and at least one maximum of the mean adhesive surface coverage of the adhesive layer in the center region can be obtained when using adhesive stripes in several ways. It is, for example, possible that one or more of such maxima are provided by one or several adhesive stripe(s) of increased width relative to the width of the strips in other areas of the adhesive layer. In the case of 3 maxima the adhesive layer thus preferably comprises two edge adhesive stripes and one adhesive stripe within the central region whereby such adhesive stripes exhibit an increased width relative to the width of the strips in other areas of the adhesive layer. Alternatively or in addition, such maxima may be obtained by providing adhesive stripes of essentially equal width in CD of the precursor laminate or activated laminate, respectively, but decreasing the width of the adhesive-free stripes between such adhesive stripes in the areas of the maxima and/or correspondingly increasing such width of the adhesive-free stripes outside the areas of the maxima respectively. Any combination of such methods can preferably be used, as well.

Stretching of the precursor is particularly effected in CD so that the elastic properties of the activated composite laminate web obtainable by stretching the precursor web can be varied and adjusted by varying and adjusting the mean adhesive surface coverage in CD.

The term "machine direction" (abbreviated as MD) as used in the present invention denotes the direction of the running, continuous web, i.e. the direction in which the web is manufactured in a continuous production process. The term "cross direction" (abbreviated as CD) denotes the direction which is substantially normal to the machine direction.

The patterned layer of adhesive between the first and, optionally, between the second non-woven web, respectively, and the elastic film can be obtained by applying a patterned layer of adhesive on the first surface of the elastic film, for example, via screen-printing or die-coating using an appropriately designed shim.

The adhesive preferably is a pressure-sensitive adhesive and, in particular, a hot-melt pressure sensitive adhesive so that the adhesive can be applied via die-coating.

The patterned adhesive layer may be preferably attached to one of the exposed surfaces of the elastic layer, and the non-woven layer is subsequently laminated onto the first exposed surface of the elastic layer. If desirable, a patterned adhesive layer may also be applied to the second exposed surface of the elastic film, and a second non-woven web is laminated onto the second exposed surface of the elastic layer. The second non-woven layer may be applied either simultaneously with or preferably subsequently to the application of the first non-woven web. Alternatively, it is also possible that one or two patterned adhesive layers, respectively, are attached to the surface(s) of the non-woven web(s) facing the elastic film, and the elastic layer is simultaneously or subsequently, respectively, attached to the two exposed surfaces of the elastic layer. It is also possible that one patterned adhesive layer is attached to a surface of the elastic film while the second adhesive layer is attached to the surface of that non-woven web facing the elastic film not bearing an adhesive layer.

The non-woven web(s) and the elastic film are attached to each other in order to provide the precursor of the composite laminate web. During such attachment the non-woven web(s) are usually contacted with each other under pressure and optionally, for example, heating in order to provide an integral precursor web. This may be accomplished, for example, by processing the non-woven web(s) and the elastic film sandwiched between the webs through an optionally heated nip.

While the adhesive pattern generated on the elastic film and/or on the non-woven web(s) prior to assembling these into the precursor web typically exhibits a pattern corresponding in a mirror-converted way to that of the shim (including, for example, sharp square leading and trailing edges in case of a shim having rectangular teeth) the pattern may be blurred out somewhat in the activatable precursor web or the activated elastic web, respectively. The extent of such blurring depends on various parameters such as processing parameters like the the nip pressure and/or temperature, the processing speed of the web in MD and/or the stretch extension applied for obtaining the elastic composite web, and/or material properties such as, for example, the visco-elasticity of the pressure sensitive adhesive.

The present inventors have investigated this effect by staining the non-stretched, stretch-activatable precursor and the stretch-activated elastic composite web, respectively, so as to make the corresponding adhesive pattern visible. Subsequently the one or more non-woven web layers of the precursor and the stretch-activated composite web, respectively, were carefully removed to measure the various stripe widths. This is described in more detail in the Example section. It was found that the adhesive pattern as initially applied, for example, via a shim is essentially maintained in the precursor and the activated composite web, respectively. It was found, for example, that in a striped adhesion pattern comprising adhesive stripes extending in MD separated by adhesive-free stripes extending in MD, the mean deviation of the width of the adhesive stripes in CD in the precursor and the activated composite web, respectively, relative to the width of the stripes as applied via a shim was less than 20 %, particularly less than 10 % and especially not more than 7.5 %. The precursor web and the activated web, respectively, preferably exhibit the same number of maxima of the local and/or mean adhesive surface coverage of the adhesive as the patterned adhesive coating originally applied to the non-woven web(s) and/or the elastic layer, respectively. In case of a pattern of the adhesive layer comprising adhesive stripes the precursor web and the activated web preferably exhibit an adhesive stripe pattern, as well. It was found by the present invention that the blurring effect was low and in any case acceptable for practical purposes when a hot-melt pressure sensitive adhesive was used.

Therefore, in the following no difference is made between the adhesive pattern as applied originally, for example, via a shim and the corresponding adhesive pattern in the activatable precursor or strech-activated composite web, respectively.

The adhesive pattern or structure used in the present invention is characterized by reciting the local adhesive surface coverage or the mean adhesive surface coverage, respectively.

The "local adhesive surface coverage", for example, in CD is defined as the ratio between the local amount of adhesive at a position in CD relative to the maximum local amount of adhesive in CD. For a pattern of parallel adhesive stripes in CD separated by stripes free of adhesives which are extending in MD, the local adhesive surface coverage is a dimensionless number assuming a value of 1 at the position of the adhesive stripes and a value of 0 in the adhesive-free stripes there between. Plotting the local adhesive surface coverage in CD as a function of the extension in CD results in a step function jumping from a value of zero in the adhesive-free stripes to a value of one at the border line between an adhesive-free stripe and an adhesive stripe and jumping from one to zero at the border line between an adhesive stripe and an adhesive-free stripe.

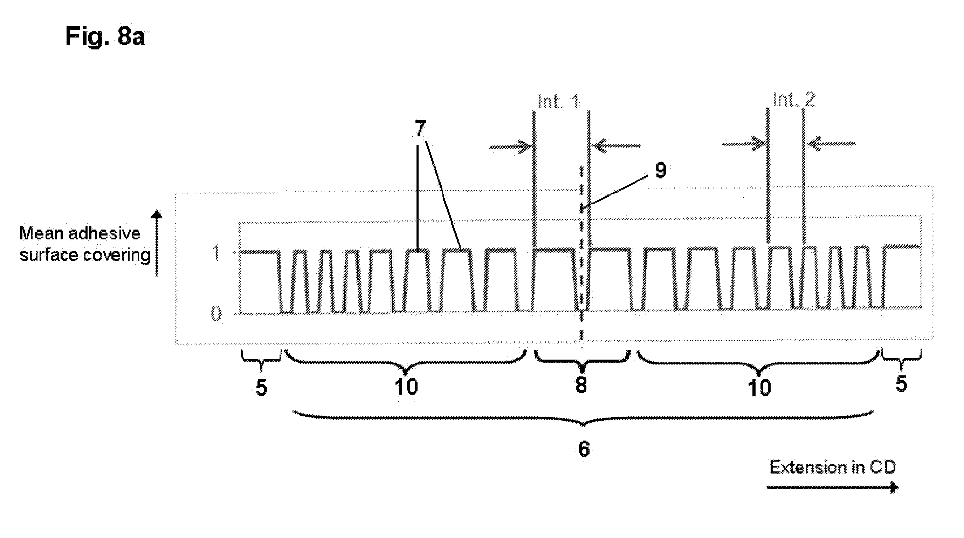

The "mean adhesive surface coverage", for example, in CD is obtained by defining intervals along the cross direction having an appropriate width over which the local area coverage is integrated to provide a mean value for each interval. This can be explained by way of an example for a pattern comprising two stripe-shaped continuously and fully coated edge regions, a continuously and fully coated adhesive center strip, and an alternating sequence of adhesive-free stripes and adhesive stripes in the areas between the edge stripes and the center stripe, respectively, which are referred to above and below as middle areas. The adhesive-free strips and the adhesive strips in the intermediate region have the same width in CD, and their width is smaller than the width of the edge stripes and the center stripe, respectively. If the width of the integration interval is chosen to be larger than the width of the adhesive stripes and the adhesive-free stripes, respectively, in the intermediate region but smaller than the width of the edge stripes and the center stripe, respectively, the smoothed curve representing the mean adhesive surface coverage exhibits relative maxima in the edge regions and the center region, respectively. The interval width has to be chosen properly. The term "smoothening" means in this connection that the plot of the mean adhesive coverage vs. extension in CD which is obtained by integration of the local adhesive surface coverage over the chosen interval and which is typically steplike, is replace with a continuous levelled curve. If the width is chosen, for example, smaller than the width of the adhesive stripes in the intermediate area the mean adhesive surface coverage still has a multitude of maxima or steps similar to the plot of the corresponding local adhesive surface coverage. If the width is chosen too large the mean area coverage may simply be constant. Yet, the skilled person will be able to define a suitable interval width in such a manner so that a smoothed plot of the mean adhesive surface coverage versus the extension in CD is a continuously varying function. This is illustrated in Fig. 8a - 8e below. For example, in a patterned adhesive layer wherein the pattern comprises adhesive stripes and adhesive-free stripes of different width in CD, the interval width should be set at least as large as the width of the widest adhesive stripe or adhesive-free stripe, whatever is higher. More preferably, the interval width should be set at least as large as the width of the widest adhesive stripe plus the width of the widest adhesive-free strip. Preferably, the interval width is in the range of between 1 mm and 3 mm, more preferably between 1.5 mm and 2.5 mm. In general, the skilled person will choose an integration interval which avoids an oscillating mean adhesive surface coverage. Optionally, a further smoothening may be necessary.

Generally, the stretch-activation of precursors of composite laminate webs having an inhomogeneous local or mean adhesive surface coverage, respectively, in cross direction results in stretch-activated composite laminate webs having an inhomogeneous elasticity behaviour in cross direction. Areas of the precursor having a high adhesive coverage result upon stretch-activation in laminates having a distinctly lower elasticity than areas having a low adhesive coverage. It has been found that additional components, such as hook tabs or fingerlift elements, can be more securely attached to areas having a maximum mean adhesive surface coverage and consequently a lower elasticity upon stretching in comparison to areas having a lower mean adhesive surface coverage.

Areas having a lower (i.e. non-maximum) mean adhesive surface coverage are by contrast relatively soft and flexible. However, such areas tend to render the activated laminate less stable in CD thereby reducing the bonding strength between the activated composite laminate and additional materials such as hook patches attached to these areas. According to the concept underlying the present invention the elastic properties in CD are adjusted and tailor-made in view of specific requirements by choosing an appropriate pattern of adhesive characterized by a variation of the mean adhesive surface coverage of the adhesive layer in CD.

The precursor web or the activated web of the present invention, respectively, preferably comprises 3, 5, 7, .... maxima of the mean adhesive surface coverage in CD. These maxima are preferably arranged essentially symmetrically relative to the center line of the web in CD. In a preferred embodiment of the precursor web or the activated web, respectively, of the present invention the adhesive layer comprises 3 maxima of the mean adhesive surface coverage in CD whereby the intermediate region comprises a maximum proximate the center liner of the web in CD. Preferably, the maximum is within ± 25 mm from the center line and more preferably within ± 20 mm from the center line. The maximum of the mean adhesive surface coverage is preferably distributed essentially symmetrical around the center line. In another preferred embodiment, the adhesive layer comprises 5 maxima of the mean adhesive surface coverage in CD whereby the intermediate region comprises 3 maxima. One of these maxima is preferably arranged proximate the center of said intermediate region, and the other two maxima are preferably arranged symmetrically proximate the middle of the distance between the edge region and the central maximum. Preferably, the central maximum is within ± 25 mm from the center of the intermediate region, more preferably within ± 20 mm from the center, and most preferably within ± 10 mm from the center. The two other maxima referred to above and below as secondary center regions, are preferably within ± 20 mm from the middle of said distance between the edge region and the central line. In a preferred embodiment the secondary center regions are essentially fully coated with adhesive so that the secondary center region forms secondary center adhesive stripes. In an especially preferred embodiment both the center region and the secondary center regions are essentially fully adhesive coated.

In a preferred method of the present invention activated webs having 5, 7, 9 .... maxima of the mean adhesive surface coverage are preferably cut into webs with 3 maxima because these can advantageously be processed in a waste-free way into a sequence of left and right ear panels applicable, for example, to the chassis of a diaper. This is illustrated, for example, in Fig.'s 4a and 4b below. In such method cutting is preferably performed so that the 3, 5, 7, ... maxima located in the intermediate region form upon cutting one maximum in each of the resulting webs with 3 maxima. Because of this, maxima located in the intermediate region preferably have a higher width in CD than the width of the maxima located in the edge regions; the width of the maxima in the intermediate region preferably is at least 1.5 times, more preferably between 1.5 and 3 times, still more preferably between 1.75 and 2.25 times and especially preferably about twice the width of the maxima in the edge regions.

It is also possible to cut the non-activated precursor web into webs with 3 maxima of the mean adhesive surface coverage but this is usually less preferred because the webs obtained upon cutting need to be activated separately from each other.

Since such preferred method always provides upon cutting webs comprising 3 maxima of the mean adhesive surface coverage in CD, the preferred embodiments of the precursor webs and activated webs, respectively, described below relate to webs comprising 3 maxima unless otherwise indicated. The person skilled in the art understands, however, that such webs can be manufactured either directly or by cutting from webs with a higher number of maxima and, in particular, from webs with 5, 7, 9, .... maxima which allow for an essentially waste-free processing as is explained in more detail below. Therefore such webs with a higher number of 5, 7, 9, .... maxima are comprised from the following specification, as well.

In a preferred embodiment of a precursor web or activated web, respectively, having 3 maxima of the mean adhesive surface coverage, the intermediate region comprises adhesive stripes extending in MD the width in CD of which increases towards the center line (or towards a position proximate the center line) of the intermediate region. The width of the essentially adhesive-free stripes between such adhesive stripes is preferably constant over the extension of the web in CD. It is possible that the width of said adhesive stripes increases continuously towards the center line or that the width of said adhesive stripes increases abruptly or stepwise towards the center line. Thus, according to such preferred embodiment the closer the adhesive stripes are to the center line the larger their width in CD.

According to another preferred embodiment the intermediate region comprises a single or several central adhesive stripe(s) with considerably increased width relative to the width of the adhesive stripes in the middle region(s) located between the edge regions and the center region or secondary center region(s), respectively. The width in CD of the adhesive stripes located in the middle region preferably is essentially constant.

It is also preferred that the intermediate region of such webs comprises adhesive stripes extending in MD including a central adhesive stripe proximate the center of the intermediate region with a substantially increased width relative to the width of the other adhesive stripes in the intermediate region. Preferably, the width of the center adhesive stripe is between 1.5 and 3 times and especially preferably about twice the width of the edge regions. For instance, the width of the center adhesive stripe is between 20 and 60 mm, preferably between 30 and 50 mm. The width of the adhesive stripes between the center stripe and the edge regions may vary. In a preferred embodiment the width of the adhesive stripes between the center stripe and the edge regions is essentially constant but such width may also increase or decrease towards the center adhesive stripe.

Preferably, the width of said edge regions is between 10 and 30 mm, more preferred between 15 and 25 mm. The edge regions are preferably formed by adhesive stripes extending in MD.

According to another preferred embodiment of a precursor web or activated web, respectively, having 3 maxima of the mean adhesive surface coverage, the center region comprises two or more adhesive stripes separated by essentially adhesive-free stripes so that one of the adhesive-free stripes is located proximate to the center line; thus the local adhesive surface coverage exhibits a minimum in the cross direction proximate the center of the intermediate region. Preferably, the minimum is within ± 10 mm from the center, more preferably within ± 5 mm from the center, and most preferably at the center. In such case the mean adhesive surface coverage may or may not exhibit a minimum in the center region, mainly depending on the width of the integration interval chosen.

In a preferred embodiment of a precursor web or activated web, respectively, having 3 maxima of the mean adhesive surface coverage, the intermediate region comprises adhesive stripes the width of which decreases towards the center (or towards a position proximate the center) of said intermediate region.

Such webs preferably exhibit in the areas between the edge region and the central adhesive stripe adhesive stripes extending in MD the width of which being in the range of between 0.5 and 5 mm, preferably between 0.8 and 2 mm. It is further preferred that the distance between adhesive stripes, i.e. the width of the adhesive-free stripes, is between 0.5 and 5 mm, preferably between 0.8 and 2 mm.

In an especially preferred embodiment having 3 maxima of the mean adhesive surface coverage in CD, the precursor web and the activated composite web, respectively, preferably comprise two fully coated edge regions and a fully coated center region. The middle region of such webs preferably comprises adhesives stripes separated by essentially adhesive-free stripes whereby the width of the adhesive stripes in the middle region is lower than the width of the edge and center adhesive stripes, respectively. Especially preferred are furthermore precursor webs and activated webs obtained therefrom by stretching which have 3, 5, 7, ... maxima in the intermediate region formed by an essentially fully coated center region and essentially fully coated secondary center regions. The center and secondary center stripes, respectively, are essentially equidistantially arranged from each other.

In another preferred embodiment, the composite laminate web comprises a second non-woven web opposite to the first non-woven web and a second layer of adhesive between the elastic film and the second non-woven web. The second layer of adhesive is preferably arranged in a pattern which may be selected independently from the pattern between the first non-woven web and the elastic film. Preferably the second adhesive layer exhibits essentially the same pattern as the first layer of adhesive whereby the first and second adhesive layer are furthermore preferably arranged in a registered fashion relative to each other so that the corresponding maxima and minima of the adhesive surface coverage are arranged opposite to each other.

The elastic layer used in the present invention exhibits elastomeric properties at ambient conditions. Elastomeric means that the material will substantially resume its original shape after being stretched. Preferably, the elastomer will sustain only a small permanent set following deformation and relaxation, which set is preferably less than 30 % and more preferably less than 20 % of the original 50 to 500 % stretch. The elastomeric material can be either made solely of elastomers or it may also comprise blends with an elastomeric phase or content so that it will still exhibit substantial elastomeric properties at room temperature. Suitable elastomeric thermoplastic polymers include block copolymers such as those known to those skilled in the art as A-B or A-B-A type block copolymers or the like. These block copolymers are described, for example, in US patents 3,265,765; 3,562, 356; 3,700,633; 4,116,917 and 4,156,673, the substance of which are incorporated herein by reference. Styrene/isoprene, butadiene or ethylene-butylene-styrene (SIS, SBS or SEBS) block copolymers are particularly useful. Generally, there are two or more blocks, at least one A-block and at least one B-block, where the blocks can be arranged in any order including linear, radial, branched, or star block copolymers. Other useful elastomeric compositions can include elastomeric polyurethanes, ethylene copolymers such as ethylene vinyl acetates, ethylene/propylene copolymer elastomers or ethylene/propylene diene copolymer elastomers. Blends of these elastomers with each other or with modifying non-elastomers are also contemplated.

Viscosity reducing polymers and plasticizers can also be blended with the elastomers such as low molecular weight polyethylene and polypropylene polymers and copolymers, or tackifying resins such as Wingtack™, aliphatic hydrocarbon tackifiers available from Goodyear Chemical Company. Tackifiers can also be used to increase the adhesiveness of an elastomeric layer to a skin layer. Examples of tackifiers include aliphatic or aromatic hydrocarbon liquid tackifiers, polyterpene resin tackifiers, and hydrogenated tackifying resins. Aliphatic hydrocarbon resins are preferred.

Additives such as dyes, pigments, antioxidants, antistatic agents, bonding aids, anti-blocking agents, slip agents, heat stabilizers, photo stabilizers, foaming agents, glass bubbles, reinforcing fiber, starch and metal salts for degradability or microfibers can also be used in the elastomeric core layer(s).

In one embodiment the elastic film preferably is a one layer film which exhibits elastic properties without stretching and which therefore does not need to be stretch-activated. Alternatively, pre-stretched elastic laminates such as those exhibiting an elastic core layer and at least one skin layer being less elastic than the core layer may be used. In such cases the elastic layer does not require activation, and the stretch-activation of the precursor mainly affects the non-woven web(s) resulting in a non-elastic deformation of such non-woven webs. One-layered elastic films when wound up in a roll tend to stick to each other so that it may not be possible to unwind such rolls without damaging the elastic films. Therefore the one-layered film may be treated, for example, with a blocking agent such as, for example, an inorganic anti-blocking agents such as talcum, chalk and/or or silicates. Alternatively the one-layered elastic film may be processed in-line so that it does not need to be wound up in roll form. Such method includes, for example, the extrusion of a one-layered elastic film with subsequent application of one or more non-woven-webs bearing an adhesive layer. If desired the extruded one-layered elastic film can be passed over a cooling roll prior to the application of the one or more adhesive layers and the corresponding non-woven webs.

Preferably, the elastic film is an activatable elastic laminate. In a preferred embodiment the elastic laminate comprises an elastic core layer and at least one skin layer which is less elastic than the core layer. Examples of such elastic laminates comprise an elastic core layer comprising the elastomeric material described above, and at least one skin layer comprising non-tacky materials or blends formed of any semi-crystalline or amorphous polymer(s) which are less elastomeric than the elastic core layer. The skin layers are preferably essentially inelastic and non-tacky, and thus will undergo relatively more permanent deformation than the core layer at the percentage by which the elastic laminate is stretched. Elastomeric materials such as olefinic elastomers, e. g., ethylene-propylene elastomers, ethylene propylene diene polymer elastomers, metallocene polyolefin elastomers or ethylene vinyl acetate elastomers, or styrene/isoprene, butadiene or ethylene-butylene/styrene (SIS, SBS or SEBS) block copolymers, or polyurethanes or blends with these materials can be used as long as the skin layers provided are essentially non-tacky and less elastomeric than the core layer. The skin layers preferably can act as barrier layers to any adhesive applied. The elastomeric materials used are present in a blend with non-elastomeric materials in a weight percent range of 0 to 70 %, preferably 0 to 50 % and more preferably 0 to 20 %. High percentages of elastomer in the skin layer(s) generally require use of antiblock and/or slip agents to reduce the surface tack and roll unwind force. Preferably, the skin layers comprise one or more polyolefin or polyolefin copolymer selected from the group consisting of polyethylene, polypropylene, polybutylene, and polyethylene-polypropylene copolymer. The skin layers, however, may also include one or more polyamides such as nylon, one or more polyesters such as polyethylene terephthalate, and suitable blends thereof.

The core:skin thickness ratio and/or the softness of the skin layer(s) are preferably controlled to allow for an essentially homogeneous activation of the activatable elastic laminate. The core:skin thickness ratio as used above and below is defined as the ratio of the thickness of the elastic core over the thickness of the at least one skin layer (if only one skin layer is present) or over the sum of the thicknesses of the two skin layers (if a second skin layer is present), respectively.

It was found that if the core:skin thickness ratio defined above is too low and/or the skin layers are too rigid the activatable elastic laminate when stretched, tends to neck macroscopically and/or form macroscopic buckles. The term "macroscopic(ally)" as used above and below means that such necked-in sections and/or buckles can be easily seen with the unaided eye. Typically the necked-in sections or the buckles have an extension of at least 1 mm.

These macroscopic neckings or buckles often only form in certain areas of the stretched elastic laminate whereas other areas of the stretched elastic laminate where the skin layers are essentially not distorted, remain flat and/or non-necked. This inhomogeneous activation behaviour of the elastic laminate imparts an unfavourable aesthetic appearance to the activated composite laminate which is not acceptable, in particular, for use in hygienic articles such as diapers. Also, the inhomogeneous activation behaviour results in a stress-strain behaviour which may be difficult to control. Once the necking and/or buckling area or areas of the elastic laminate have been stretched to an extent so that the force applied may be sufficient to induce necking and/or buckling in other area(s), the stress-elongation curve may exhibit further peaks, and further stretching of the elastic laminate may be zippy.

The core:skin thickness ratio and/or the softness of the skin layers of the elastic laminate need to be selected so that the skin layer(s) when stretched beyond their elastic limit and relaxed with the core form a microstructured surface texture. Microstructure means that the surfaces of the skin layers of the elastic laminate contain microscopic peak and valley irregularities, folds or other microscopic surface structure elements which are large enough to be perceived by the unaided human eye as causing increased opacity over the opacity of the elastic laminate before microstructuring, and which irregularities are small enough to be perceived as smooth or soft to human skin. Magnification of the irregularities is required to see the details of the microstructured texture.

The core:skin thickness ratio and/or the softness of the skin layers of the elastic laminate furthermore needs to be selected so that the elastic laminate can be stretched essentially homogeneously as indicated by the absence of any macroscopic buckles and/or an essentially homogeneously increased opacity of the elastic laminate as compared to the initial opacity before stretching the elastic laminate.

The core:skin layer thickness ratio useful in the present invention preferably is at least 6:1, more preferably at least 7:1 but less than 1,000:1. Most preferably such ratio is between 7:1 and 25:1.

The precursor web and the activated web of the present invention, respectively, comprise one or two nonwoven webs which are attached to the exposed surfaces of the elastic layer; in the preferred embodiment where the elastic layer is formed by an elastic laminate comprising an elastomeric core layer and one or two skin layers, the one or two non-woven webs are attached to such one or two exposed skin layers, respectively. The nonwoven web used in the present invention preferably comprises carded non-woven materials.

The non-woven webs used in the present invention are preferably made from pre-bonded staple fiber webs having an elongation at break in cross-direction of at least 100 %, more preferably of at least 120 % and especially preferably of at least 150 %. The pre-bonded staple fiber webs suitable in the present invention include air-laid, wet-laid and carded nonwoven webs with carded nonwoven webs being preferred.

Carded non-woven webs are preferably made from separated staple fibers which fibers are sent through a combing or carding unit which separates and aligns the staple fibers in the machine direction so as to form a generally machine direction-oriented fibrous nonwoven web. If desired, the degree of machine direction orientation may be reduced and/or adjusted by randomizers.

Once the carded web has been formed, it is then bonded by one or more of several bonding methods. One bonding method is powder bonding wherein a powdered adhesive is distributed through the web and then activated, usually by heating the web and adhesive with hot air. Another bonding method is pattern bonding wherein heated calendar rolls or ultrasonic bonding equipment are used to bond the fibers together.

Pre-bonded staple fiber webs useful in the present invention preferably exhibit a localized discontinuous bond pattern such as, for example, a multiplicity of discrete thermal bond points throughout the web.

The staple fiber webs used in the present invention are preferably pre-bonded. Non-pre-bonded nonwoven webs tend to form a fuzzy or curly surface upon stretch activation because the ends of unbonded individual fibers may stick out of the surface. This is not desirable and/or acceptable, in particular, for baby hygiene products such as baby diapers where the baby may try to rip off such lose fibers and may swallow them.

In a preferred embodiment of the present invention the precursor web or the activated web of the present invention pre-bonded nonwoven webs are attached to the elastic layer by the one or two patterned adhesive layers described above. The pre-bonded bonding pattern of the nonwoven web is therefore completely separate from and independent of the bonding between the nonwoven web(s) and the elastic layer which is effected by said patterned adhesive layer(s). Although the present inventors do not wish to be bound by such explanation it is speculated that such independent and separate pre-bonding pattern of the nonwoven web provides a multiplicity of joints which imparts on the one hand a favourable stretching behaviour and a high elongation at break to the nonwoven web while maintaining on the other hand a sufficient integrity of the nonwoven web.

The pre-bonded nonwoven webs suitable in the present invention preferably exhibit a bonding area of at least 8 %, more preferably of at least 9 % and especially preferably of at least 9.5 % with respect to the surface of the nonwoven web. If the bonding area of the nonwoven web is less than 8 % with respect to the surface of the nonwoven web the mechanical integrity of such web tends to be insufficient, in particular, for hygienic applications.

The pre-bonded staple fiber nonwoven webs which are preferred in the present invention are anisotropic because they are distinctly stronger in the machine direction in which the fibers are oriented by the combing or carding step as compared to the cross-direction. Generally, with increasing bonding area of the pre-bonded nonwoven web, its tensile strength in the cross-direction will increase and the elongation at break will decrease. In the present invention, the bonding area of the pre-bonded nonwoven web and the average degree of orientation of the fibers in the machine direction (which can be adjusted by using randomizers as was described above) are preferably selected so that the ratio of the tensile strength at break in the machine direction over the tensile strength of break in the cross-direction is preferably between about 5:1 to 7:1 and more preferably between about 5.3:1 to 6.7:1.

It was furthermore found that the pre-bonded nonwoven webs useful in the present invention preferably exhibit a bonding area of not more than 22 %, more preferably of less than 18 % and especially preferably of less than 15 % with respect to the surface of the nonwoven web. If the bonding area of the pre-bonded nonwoven web is higher than 22 % with respect to its surface, the nonwoven web tends to be too strong in the cross-direction and the elongation at break in the cross-direction tends to be too low.

The bonding area of the pre-bonded nonwoven web preferably is between 9 and 18 % and more preferably between 9 and 15 % with respect to the surface of the nonwoven web.

The staple fiber nonwoven webs suitable in the present invention preferably comprise one or more fibers selected from the group consisting of natural or synthetic fibers selected from cotton, rayon, polyolefins including polyethylene and polypropylene, polyamides including nylon, polyesters including polyethylene terephthalate, aramids and blends thereof. Polyolefin based fibers are generally preferred.

The staple fiber nonwoven webs suitable in the present invention preferably have an average staple length in the machine direction of between 30 and 80 mm and more preferably of between 30 and 60 mm.

The patterned adhesive layers(s) of the precursor web and the activated web of the present invention, respectively preferably comprise adhesives which are activatable by pressure, heat or combination thereof. Especially preferred are hot-melt pressure sensitive adhesives. Suitable adhesives include those based on acrylate, rubber resin, epoxies, urethanes or combinations thereof. The patterned adhesive layer may be applied by various techniques including, for example, die-coating or screen printing methods. Adhesive layers comprising adhesive stripes which are separated by essentially adhesive-free stripes are preferably attached by die-coating using an appropriately designed shim which may be sandwiched between an upper and a lower half of the die, respectively, as is described, for example, in US 5,685,911. The design of the shim preferably is mirror-inverted to the adhesive pattern, i.e. the shim preferably is a grid having slits where adhesive stripes are required and bars to produce essentially adhesive-free stripes therebetween, respectively.

Useful adhesives according to the present invention include all pressure-sensitive adhesives. Pressure-sensitive adhesives are well known to possess properties including: aggressive and permanent tack, adherence with no more than finger pressure, and sufficient ability to hold onto an adherent. Examples of adhesives useful in the invention include those based on general compositions of polyacrylate; polyvinyl ether; diene rubber such as natural rubber, polyisoprene, and polybutadiene; polyisobutylene; polychloroprene; butyl rubber; butadiene-acrylonitrile polymer; thermoplastic elastomer; block copolymers such as styrene-isoprene and styrene-isoprene-styrene (SIS) block copolymers, ethylene-propylene-diene polymers, and styrene-butadiene polymers; poly-alpha-olefin; amorphous polyolefin; silicone; ethylene-containing copolymer such as ethylene vinyl acetate, ethylacrylate, and ethyl methacrylate; polyurethane; polyamide; epoxy, polyvinylpyrrolidone and vinylpyrrolidone copolymers; polyesters; and mixtures or blends (continuous or discontinuous phases) of the above. Additionally, the adhesives can contain additives such as tackifiers, plasticizers, fillers, antioxidants, stabilizers, pigments, diffusing materials, curatives, fibers, filaments, and solvents. Also the adhesive optionally can be cured by any known method.

Pressure-sensitive adhesives including, in particular, hotmelt pressure-sensitive adhesives are preferred in the present invention because of their general visco-elastic properties which translate into favourable stretching properties.

It is particularly preferred that the precursor web composite laminate of the present invention comprises in each case a single patterned adhesive layer between the elastic film and the respective non-woven web. In other words, according to the present invention there is no separate and additional stiffening adhesive necessary.

In a preferred embodiment of the present invention the elastic film preferably extends essentially over the whole width in CD of the precursor web or the activated composite laminate web, respectively.

The activatable precursor of the composite laminate web of the present invention can be activated essentially without distorting and/or rupturing the non-woven layer using, for example, the width stretching device disclosed, for example, in US 5,043,036.

Such device which is also referred to as diverging disks stretching device, comprises two circular pulleys or disks mounted on a frame for rotation about their axes with the axes being oriented to position portions of the peripheral surfaces of the pulleys at a close spacing at a first location relative to the frame, and to position portions of the peripheral surfaces of the pulleys at a far spacing significantly greater than the close spacing at a second location relative to the frame and diametrically across the pulleys from the first location. The device also comprises two continuous flexible belts. The belts and the pulleys have interacting guide means extending longitudinally along the belts and circumferentially around the peripheral surfaces of the pulleys for maintaining the belts in circumferential alignment around the peripheral surfaces of the pulleys. These interacting guide means are preferably provided by the peripheral surfaces of the pulleys having a plurality of spaced circumferentially extending ridges with recesses between the ridges, and the belts having along one side a plurality of longitudinally extending spaced ridges with recesses between the ridges. The ridges on the belts are adapted and aligned to enter the grooves in the pulleys, and the ridges on the pulleys are adapted and aligned to enter the grooves in the belts. The belt is mounted on the frame for movement along predetermined paths including clamping path portions with the interacting ridges and grooves on the belts and pulleys, respectively, in engagement from an inlet position adjacent said first location to an outlet position adjacent said second location with the belts being biased towards the pulleys.

In such device the two opposing edge areas of the activatable precursor of the composite laminate web extending in the machine direction, are clamped at the inlet position to the peripheral surfaces of the pulleys by the belts, and the activatable composite laminate web is stretched to widen its width in the cross-direction as the pulleys rotate during the movement of the composite laminate web from the inlet position to the outlet position. The activated composite laminate being released from the interacting guide means at the outlet position of the device may be wound into a roll in its relaxed state.

The extent of cross-directional stretching provided by such diverging disks stretching device can be varied by varying the distance between the portions of the peripheral surfaces of the pulleys at the first and second location, respectively. If desired the cross-directional stretch ratio can be continuously varied over a wide range of, for example, between 10 and 500 %, more preferably between 20 and 300 % and especially preferably between 40 and 250 %. The elongation ratio of the width to which the precursor is stretched in the CD during the stretching process to the original width corresponds to the sum of such stretch ratio plus 100%.

The rate at which the activatable composite laminate is stretched can be varied by varying the rotation speed of the pulleys and/or the diameter of the pulleys. For a given rotation speed of the pulleys, larger diameter pulleys effect a slower rate of stretching than smaller diameter pulleys. If desired the stretching rate provided by the diverging disks stretching apparatus can be varied essentially continuously in a broad range of preferably from 10 m/min to 600 m/min. Preferably, the stretching rate is adjusted between 50 m/min and 500 m/min, and more preferably between 100 m/min and 400 m/min.

A preferred diverging stretching device is schematically illustrated by Fig. 1 and 2 of US 5,043,036, and it is described in some detail in col.4, line 10 to col. 8, line 13 of this reference. Therefore Figs. 1 and 2 and the passage of US 5,043,036 specified are incorporated by reference into this specification.

While the precursor of the activated composite laminate web or the activated web, respectively, according to the present invention may be provided in any form or packaging, it is preferred to wind such webs up in the form of a roll. Such a roll can advantageously be premanufactured and delivered to, e.g., a diaper manufacturer for the efficient production of diapers. Rolls of precursor webs or activated webs, respectively, which exhibit 3 maxima of the mean adhesive surface coverage in CD whereby two maxima are arranged in the edge region and the third maximum is proximate to the center of the intermediate region or the center line of the web in CD, respectively, are preferred. A sequence of left and right panels can be cut from such webs in an essentially waste-free manner as is exemplified, for example, in Fig.'s 4a and 4b below. Rolls of activated web are often preferred because no activation step needs to be included at the diaper manufacturing site prior to cutting the web into such sequence of individual left and right panels. Especially preferred are rolls of precursor webs or activated webs, respectively, where the adhesive layer comprises adhesive stripes whereby the adhesive strips forming the edge regions and the adhesive stripes proximate the center line of the web have a larger width in comparison with the other adhesive stripes in the intermediate region. The adhesive stripe or stripes proximate the center region preferably have a larger width in CD than the adhesive stripes forming the edge regions.

The present invention is further directed to a method of manufacturing an activatable precursor of a composite laminate web and the corresponding activated composite laminate web, respectively. In a first step of this method an elastic film having first and second surfaces may be provided. Then a first layer of adhesive is attached to the first surface of the elastic film. The first adhesive layer exhibits an adhesive pattern so that the mean adhesive surface coverage measured in the cross direction comprises at least 3 maxima wherein each edge region exhibits at least one maximum. The further maximum is arranged proximate the center of the intermediate region or the center line of the web, respectively. In another preferred embodiment the intermediate region comprises 3, 5, .... whereby the distances between the edge regions and the adjacent maxima and between adjacent maxima, respectively, are preferably essentially equal. This means that in case of 1 maximum in the intermediate region, this is preferably arranged proximate the center line of the web; in case of 3 maxima in the intermediate region, one maximum is arranged proximate the center line of the web, and the other two maxima are arranged proximate the middle of the distance between the edge regions and the central maximum, and so on. In a preferred embodiment the patterned adhesive layer preferably comprises adhesive stripes extending in CD which are separate by stripes essentially free of adhesive. The maxima in the edge regions and proximate the center line (in the case of 1 maximum in the intermediate region) are preferably formed by adhesive stripes which are wider in CD than the other adhesive strips in the intermediate region. In case the maxima in the edge regions and proximate the center of the intermediate region or the center line of the web, respectively, are formed by two or more adhesive strips, these adhesive strips may be wider than the other adhesive strips in the intermediate region and/or the width of the one or more adhesive-free strips in the edge regions or in the area proximate the center may be narrower than the other adhesive-free strips in the intermediate region.

Then a non-woven web is provided and attached via said first patterned adhesive layer to the elastic layer.

According to a preferred embodiment the method further comprises the steps of providing a second patterned adhesive layer on the second surface of the elastic film opposite to its first surface. The pattern of the second adhesive layer can be selected independently from the pattern of the first adhesive layer but preferably the pattern of both adhesive layers is essentially the same and arranged in a registered fashion so that corresponding maxima, middle regions etc. are directly opposite each other. Then a second non-woven web is provided and attached via said second patterned adhesive layer to the second surface of the elastic layer. If desired, the first and second adhesive layers can also be applied essentially simultaneously to the two surfaces of the elastic layer; likewise, the two non-woven webs can also be attached simultaneously.

In another method of the present invention which is preferred, a non-woven web is provided in the first step and a first patterned adhesive layer is applied to the surface of the non-woven web which is attached to the first surface of the elastic layer in a subsequent step. Likewise, a second non-woven layer can be provided with a second patterned adhesive layer and be attached to the second surface of the elastic layer.

Alternatively, it is also possible that an activatable precursor web exhibiting two non-woven webs is obtained by applying one patterned adhesive layer onto one of the surfaces of the elastic layer and the second patterned adhesive layer onto the surface of a second nom-woven web to be applied to the other surface of the elastic layer.

The activatable precursor web is subsequently stretch-activated, for example, in a tenter stretch activation apparatus disclosed, for example, in US 2004/0,115,411, or in a diverging disks stretching apparatus disclosed above. It is also possible to stretch-activated the precursor web by the so-called ring-rolling technique.

If the activated web has more than maxima of the mean adhesive surface coverage in CD, it is preferably cut into activated webs each comprising three maxima of the mean adhesive surface coverage and wound up in the form of a roll. It is also possible that the activatable precursor (or appropriately cut precursor webs, if applicable, each comprising three maxima of the mean adhesive surface coverage) are wound into the form of a roll.

The activated composite laminate web according to the present invention provides several advantages. Due to the inhomogenous distribution of the mean adhesive surface coverage in CD the activated composite laminate web exhibits a variation of the elastic properties in CD whereby areas exhibiting maxima in the mean adhesive surface coverage exhibit a lower elasticity in comparison to areas between the maxima of the mean adhesive surface coverage. The areas with a lower elasticity represent well-defined and predetermined adhesive bonding areas where additional components such as, for example, a mechanical hook patch or a fingerlift element can be bonded via an adhesive layer to the activated laminate web. In such areas the adhesive bonding strength between the composite web and the additional component is higher than in areas with a higher elasticity. By the same token, the activated web offers an increased elasticity in the areas outside the maxima of the mean adhesive surface coverage thus increasing the overall elasticity of the web. This combination of properties and the ability to tailor-make an elasticity profile in CD by varying the pattern of the adhesive layer(s) is highly desirable for applications on disposable products such as diapers. For instance, as mentioned above, an activated composite laminate web of the invention comprising 3 maxima of the mean adhesive surface coverage may be cut in a sequence of left and right side panels which each exhibit a soft and flexible center region and stiffer edge regions which provides good adhesive bonding areas. One of the edge regions of a panel may thus be adhesion-bonded, for example, to the diaper chassis while a hook fastening tab is applied to the other edge region.

Alternatively or in addition, the pattern of adhesive can be used to design the stretching characteristics of the activated composite laminate web. For example, the mean adhesive surface coverage can be optimized in view of the geometry of the panel so as to achieve a near-constant force when expanding or stretching the panel on the diaper. In conclusion, the use of an adhesive pattern in the precursor of the present invention in combination with the subsequent stretch-activation is a powerful tool to adjust and tailor-make the elastic properties of a composite laminate in a simple and cost-efficient manner.

### Brief description of the figures

The construction and advantages of the composite laminate web of the present invention will become more apparent from the description of preferred embodiments of the present invention with reference to the following figures, in which:
- Figure 1: is a schematic cross-sectional view of a composite laminate web according to the prior art.
- Figure 2: schematically shows a step of the method of manufacturing diaper panels from the composite laminate web of Fig.1.
- Figure 3a: is a schematic cross-sectional view of a preferred embodiment of a precursor of a composite laminate web or the corresponding activated web, respectively, according to the present invention or the corresponding activated web, respectively.
- Figure 3b: is a schematic cross-sectional view of another preferred embodiment of a precursor of a composite laminate web or the corresponding activated web, respectively, according to the present invention.
- Figs. 4a and b: schematically show two steps of the method of manufacturing diaper panels from the precursor or the composite laminate web according to Fig. 3a, respectively.
- Figure 5a: is a schematic cross-sectional view of another preferred embodiment of a precursor of a composite laminate web or the corresponding activated web, respectively, according to the present invention.
- Figure 5b: is a cross-sectional view of the precursor web or activated composite laminate web, respectively, of Figure 5a after cutting.
- Figure 6a: is a cross-sectional view of another preferred embodiment of a precursor of a composite laminate web or the corresponding activated web, respectively, according to the present invention.
- Figure 6b: is a cross-sectional view of the composite laminate web of Figure 6a after cutting.
- Figure 7a: shows a hysteresis curve of a piece of the activated composite laminate web of Fig. 3a which was cut so as to include the center region.
- Figure 7b: shows a hysteresis curve of a piece of the activated composite laminate web of Fig. 3a which was cut from the middle region of such web
- Figure 7c: shows a comparison of the hysteresis curves of Figures 7a and Figure7b.
- Figure 8a: schematically shows the local adhesive surface coverage of the adhesive layer across the extension in CD of the embodiment shown in Figure 5a.
- Figure 8b: schematically shows the mean adhesive surface coverage of the embodiment shown in Figure 5a calculated from the local area coverage shown in Figure 8a by using a first interval Int 1 with a first width.
- Figure 8c: schematically shows the mean adhesive surface coverage of the embodiment shown in Figure 5a calculated from the local area coverage shown in Figure 8a by using a second interval Int 2 having a second width.
- Figure 8d: schematically shows the local adhesive surface coverage of the adhesive layer across the extension in CD of the embodiment shown in Figure 3a.
- Figure 8e: schematically shows the mean adhesive surface coverage of the embodiment shown in Figure 3a calculated from the local area coverage shown in Figure 8d by using a third interval Int 3 having a third width.

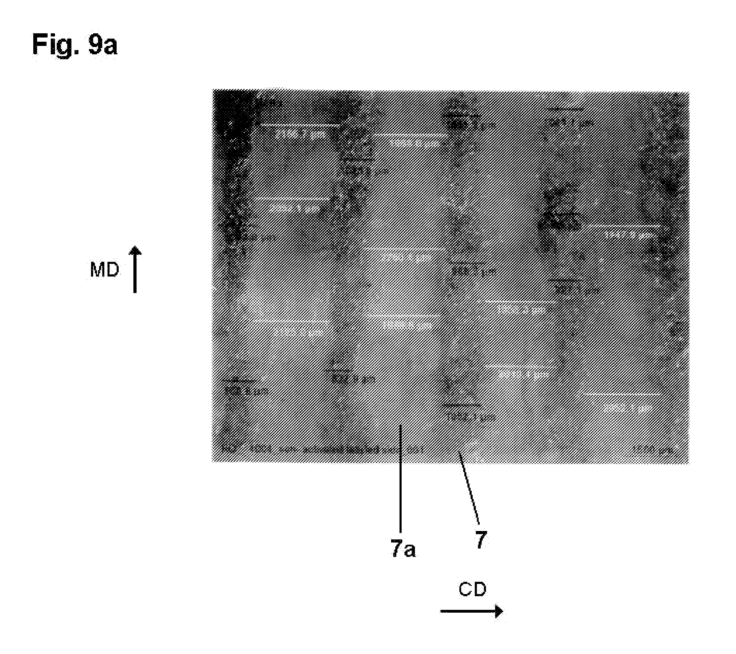

- Figure 9a: shows the pattern of the adhesive layer in the middle region of the non-activated precursor web of Example 2.

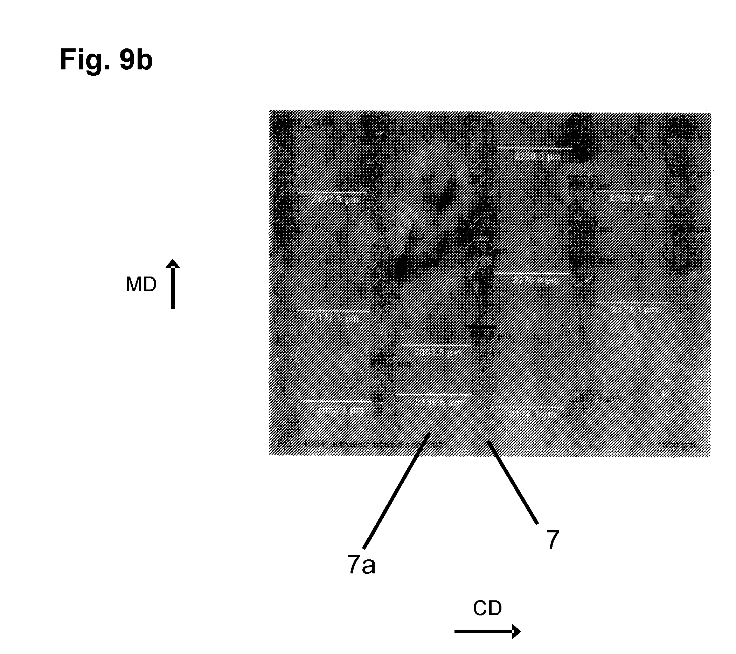

- Figure 9b: shows the pattern of the adhesive layer in the middle region of the activated composite laminate web of Example 2.

### Detailed description of the figures

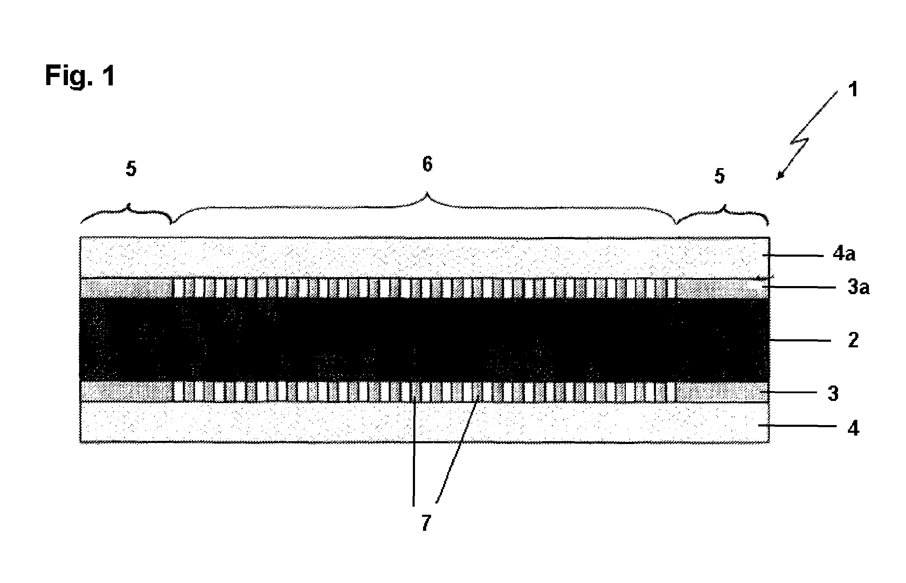

Fig. 1 shows a composite laminate web 1 of the prior art disclosed in WO 2006/124337 A1. The composite laminate web of Figure 1 comprises an elastic film 2, first and second layers of adhesive 3 and 3a, and first and second non-woven webs 4 and 4a. In an intermediate region 6, the first and second layers of adhesive 3 and 3a are structured in a pattern of adhesive stripes 7. However, at the edges of the composite laminate web the first and second layers of adhesive 3 and 3a comprise edge regions 5, which are continuously, i.e. fully, covered with adhesive. Those edge regions 5 have a stripe shape, as well, but their width in CD is larger than the width of the adhesive stripes 7 in the intermediate region 6. Therefore the edge regions provide zones of less flexibility, but increased shear strength. Thus, if a fastening tab is attached to the edge of the composite laminate web strengthened by the continuously, fully coated edge region 5, there will be a reduced risk of ripping off the fastening tab from the composite laminate web. Similarly, if the edge 5 of the composite laminate web 1 shown in Figure 1 is attached to the chassis of a diaper, the connection between the composite laminate web, i.e. the diaper ear, and the diaper chassis will be more stable.

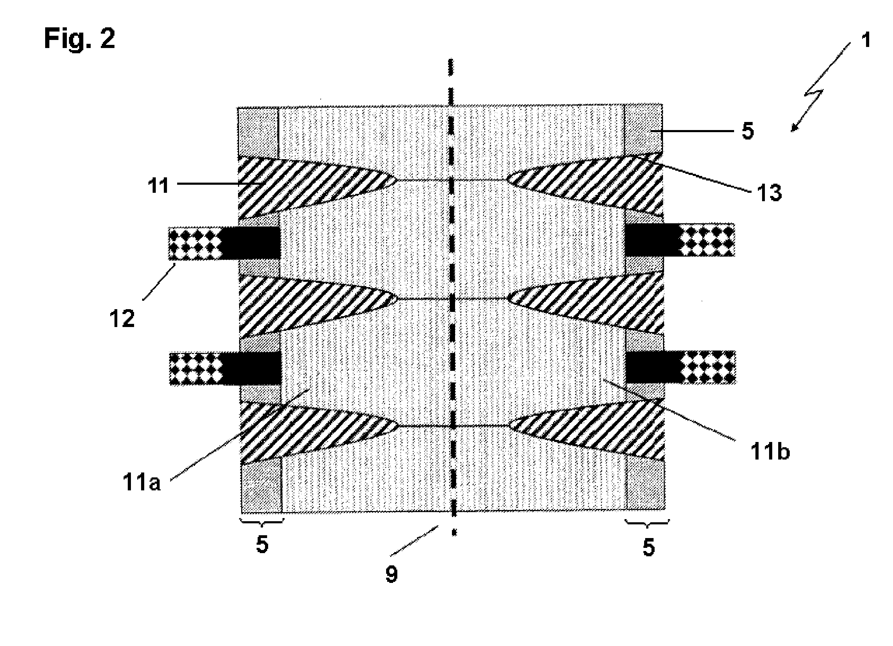

However, the solution shown in Figure 1 is only appropriate, if the composite laminate web shown in Figure 1 is used to provide single diaper panels obtained when cutting the web in CD. Yet, when manufacturing diaper panels, it is often preferred to manufacture pairs of panels which can be attached to the left and right edge, respectively, of the diaper chassis. Fig. 2 illustrates the application of such process which is known, e.g., from WO 2008/036706 A1, to the web of Fig. 1. In order to provide an appropriate shape of the diaper panels, a cut-out 11 is punched out or stamped out. This is done symmetrically on either side of the composite laminate web 1. The cut-out represents waste and needs to be discarded. Furthermore, fastening tabs or hook tabs 12 are attached at the edge regions 5 of the composite laminate web 1. Finally, the composite laminate web 1 is cut along a center line 9. Afterwards, the separate pieces 11a and 11b of the composite laminate web 1 with the hook tabs 12 attached thereto can be used as diaper panels and may, e.g., be attached to the two sides of a diaper chassis.

Apparently, if the diaper panels are manufactured as shown in Figure 2, only the connection between the hook tab 12 and the composite laminate web 1 is strengthened due to the continuous edge region 5. Yet, the opposite side of the diaper panel, which is located proximate the center line 9, comprises a pattern of stripes 7 of adhesive which is not strong enough to take up the force between diaper ear and diaper chassis when the diaper ear is stretched by pulling at the hook tab.

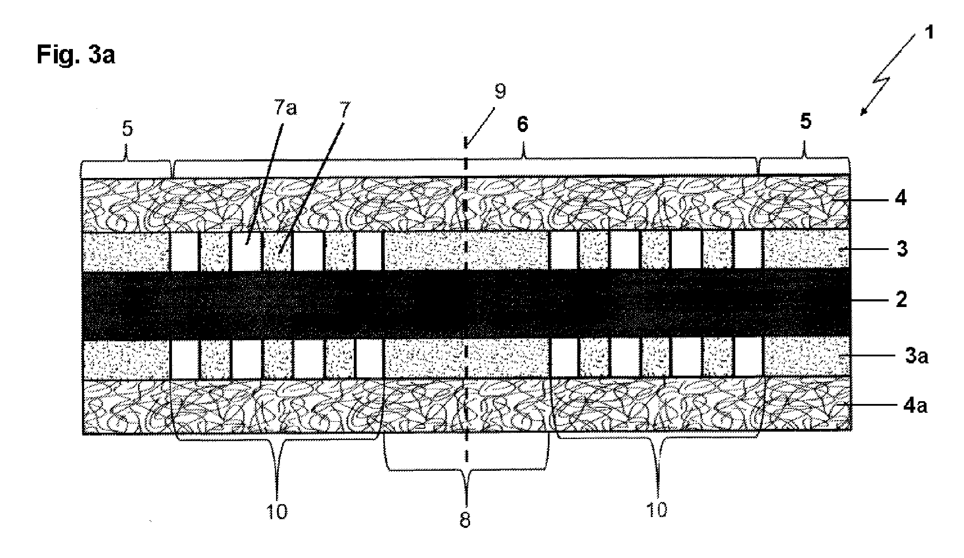

This problem is overcome by the embodiment of the stretch-activatable precursor composite laminate web or the activated composite laminate web, respectively, according to the present invention which is shown in Figure 3a. Since the structure of the precursor web and the activated web is essentially identical the schematic cross-sectional views of Fig.'s 3a, 3b, 5a, 5b, 6a, 6b and the schematic top views of Fig.'s 4a and 4b represent both the precursor web and the activated web, respectively. The precursor composite laminate web 1 shown in Figure 3a differs from the known composite laminate web shown in Figure 1 in that the pattern of stripes of adhesive 7 in the first and second layers of adhesive 3 an 3a comprises an adhesive center region 8 whose width is considerably larger than the width of the other adhesive stripes 7. Preferably, the width of the adhesive center region 8 is between 1.5 and 2.5 times and especially preferably about twice the width of the edge regions 5. Both the center region 8 and the edge regions 5, respectively, are continuously, i.e. fully coated with adhesive. The precursor web of Fig. 3a is preferably activated by gripping the two edge regions 5 with the clamps or jaw of a stretching apparatus and stretching the precursor to a desired extension in the CD. It was surprisingly found that the center region 8 exhibits upon stretching a distinctly lower elasticity in comparison to the elasticity obtained in the two middle regions 10.

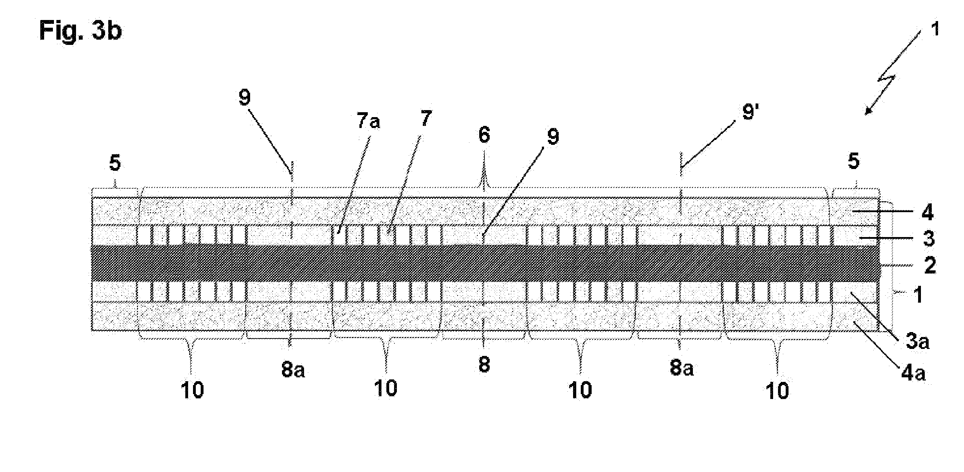

Fig. 3b shows another embodiment of the precursor of a composite laminate web or an activated composite laminate web, respectively, of the present invention which is similar to the construction of Fig. 3a but comprises in addition to the center adhesive stripe 8 two further secondary center adhesive stripes 8a (i.e. adhesive stripes arranged in the secondary center regions 8a) which are each arranged in the middle region 10 between the adjacent edge region 5 and the center stripe 8. The precursor web of Fig. 3b exhibits 4 middle regions 10 exhibiting adhesive stripes 7 being less wide in CD than the coated edge regions 5, the center adhesive stripe 8 and the secondary center adhesive stripes 8a, respectively, being continuously, fully adhesive-coated. The precursor web of Fig. 3a is preferably activated by gripping the two edge regions 5 with the clamps or jaw of a stretching apparatus and stretching the precursor to a desired extension in the CD. It was surprisingly found that the center region 8 and the secondary center regions 8a exhibit upon stretching a distinctly lower elasticity in comparison to the elasticity obtained in the four middle regions 10. Alternatively, the precursor web can first, i.e. prior to the activation step, be cut into 4 sub-webs each comprising two edge regions 5 and a center region 10 which are activated subsequebt to the cutting step.

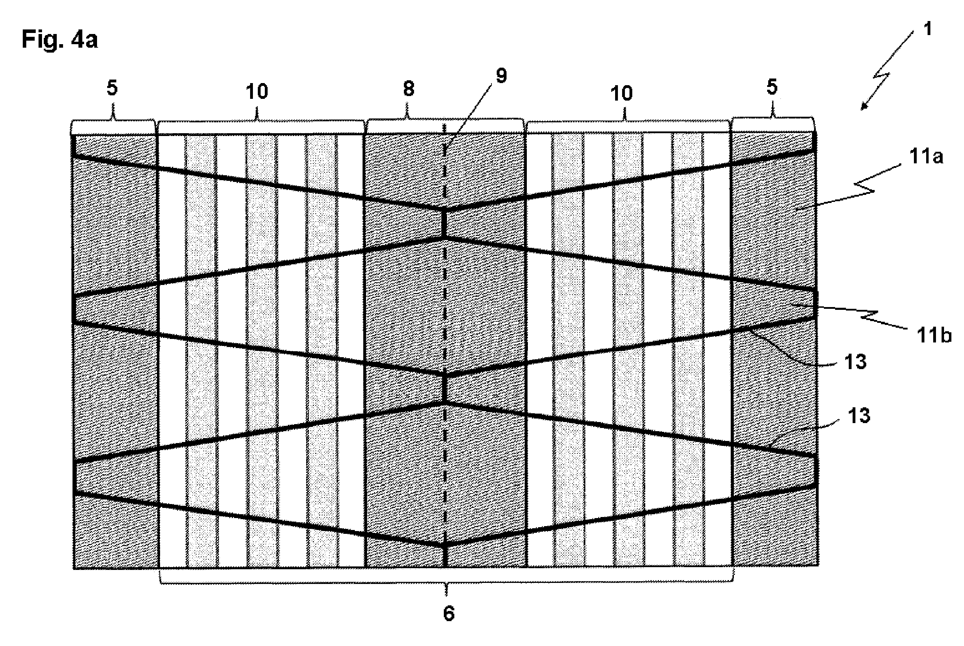

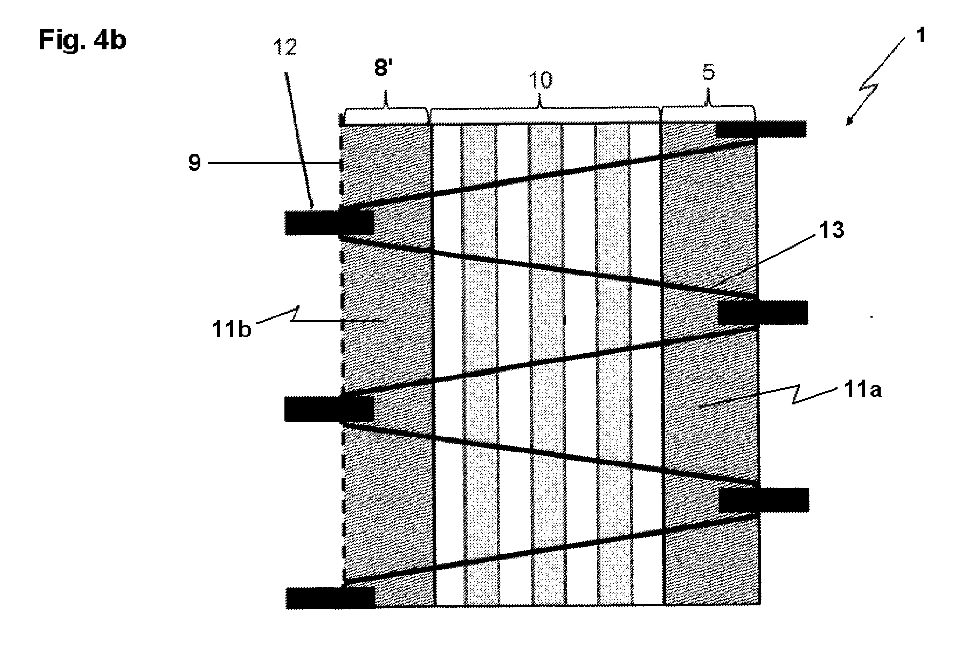

Fig.'s 4a and 4b are schematic top views illustrating the processing of the activated composite laminate web obtained by stretching the precursor of Fig. 3a. The activated composite laminate web 1 is cut along the center line 9 thereby providing two identical activated composite laminate webs (see Fig. 4b) comprising a first edge region 8' (obtained from the center area of the web of Fig. 3a), a second edge region 5 and the middle region 10.The two edge regions 5, 8' of the web of Fig. 4b exhibit a lower elasticity than the middle region 10 By applying the cut lines 13 the web of Fig. 4b can be cut into a sequence of left and right panels 11a, 11b which each exhibit a trapezoidal form. The wider low-elasticity edge of a panel can advantageously be adhesively bonded, for example, to the chassis of a diaper whereas a closure tape tab 12 bearing a mechanical hook patch or a hook patch may be attached, for example, to the narrower low-elasticity edge of the side panel.

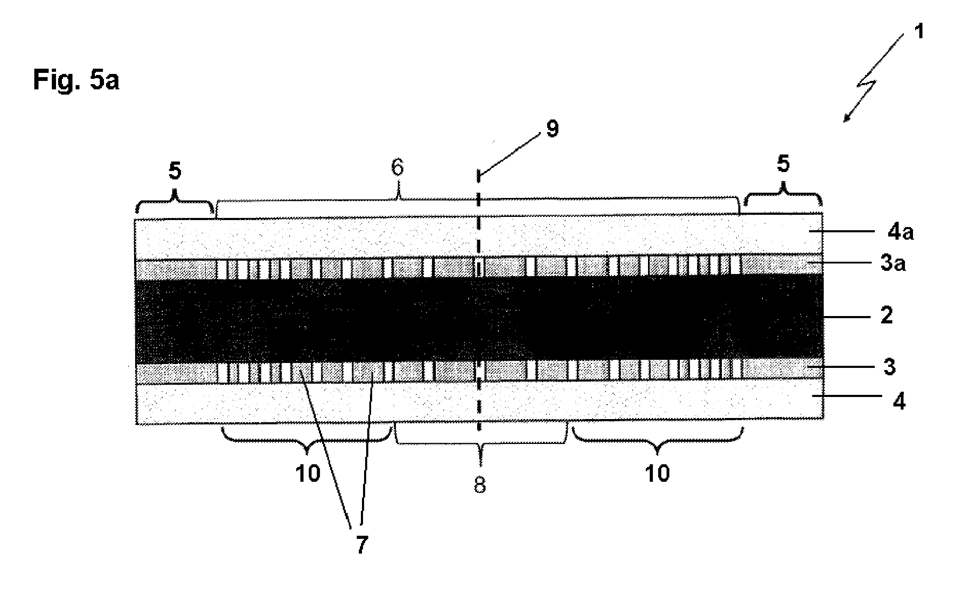

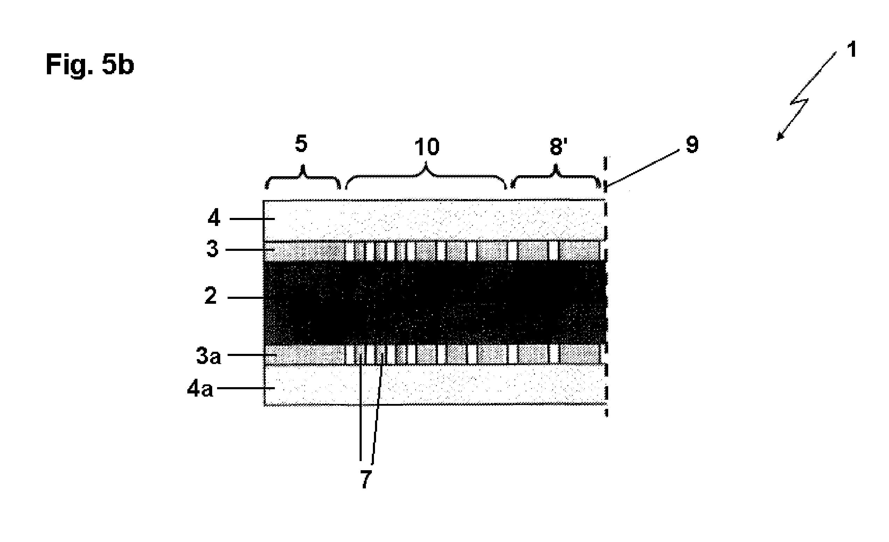

Instead of providing a single adhesive center stripe 8 of particularly large width as shown in Figure 3a, it is also possible that the width of the adhesive stripes 7 increases towards the center line 9 as is shown in Figure 5a. The precursor web or activated web, respectively, of Fig. 5a comprises an elastic layer 5 which is covered on each side by a non-woven web 4, 4a which are bonded by patterned adhesive layers 3, 3a. While the edge regions 5 are continuously, fully coated the centre region 8 exhibits several adhesive stripes 7 which are wider in CD than the adhesive stripes in the middle regions 10. It was surprisingly found that the center region 8 exhibits upon stretching a distinctly lower elasticity in comparison to the elasticity obtained in the two middle regions 10. Fig. 5b shows that the stretch-activated web is cut along the center line 9 to provide two webs exhibiting less elastic edge regions 5 and 8' and a middle region 10 with higher elasticity. The web of Fig. 5b can then be processed as has been described for the web of Fig. 4b above.

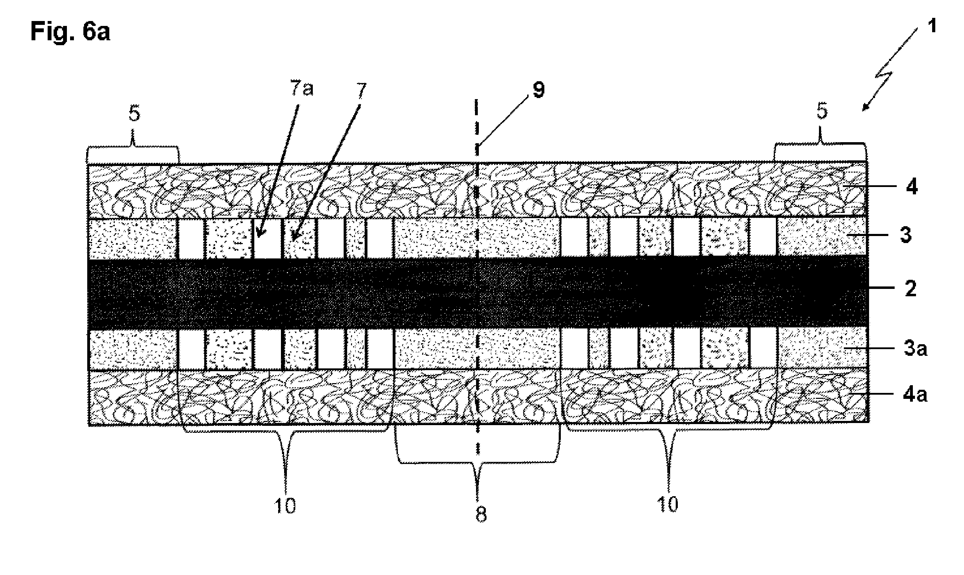

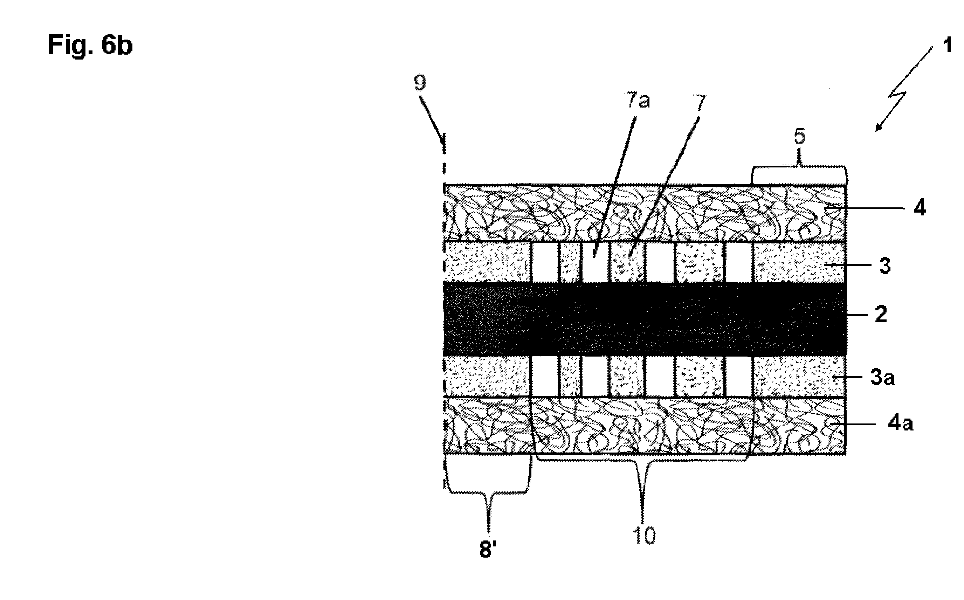

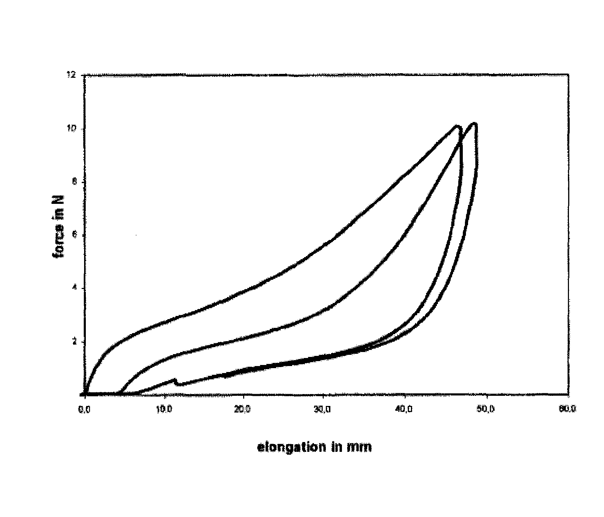

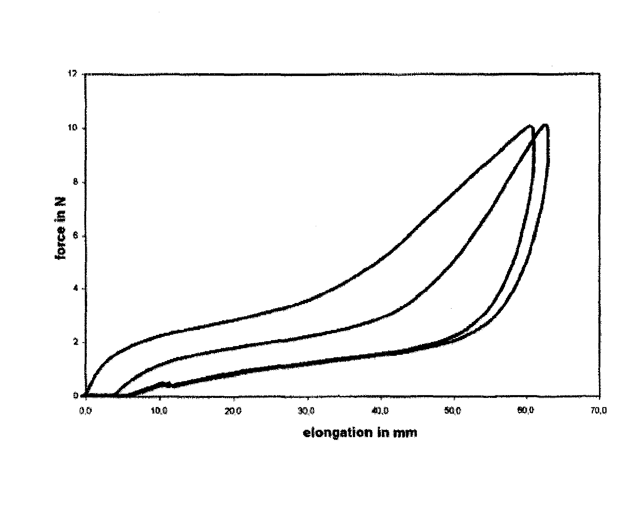

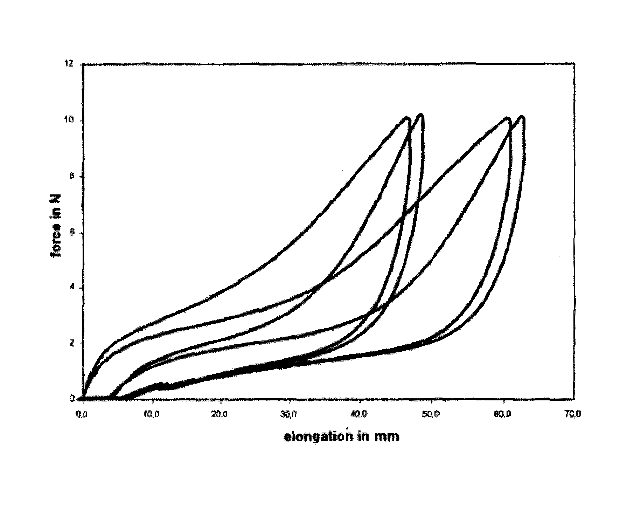

Another preferred embodiment of a precursor of a composite laminate web or an activated web, respectively, according to the present invention is shown in Figure 6a. The embodiment shown in Figure 6a is similar to the embodiment shown in Figure 3a. However, while in the embodiment of Fig. 3a the adhesive stripes 7 in the middle regions 10 are of essentially constant width, the width of the adhesive stripes 7 of the embodiment shown in Figure 6a decreases towards the center region 8. The edge regions 5 and the center region 10 are fully coated with adhesive. Fig. 7b shows that the stretch-activated web obtained by stretch-activating the precursor web of Fig. 6a, is cut along the center line 9 to provide two webs exhibiting less elastic edge regions 5 and 8' and a middle region 10 with higher elasticity. The web of Fig. 6b can then be processed as has been described for the web of Fig. 4b above.

Fig.'s 7a - 7c are described in Example 1 below.

Figure 8a schematically shows the local adhesive surface coverage of the precursor web shown in Figure 5a. The local adhesive surface coverage varies in a stepwise fashion between 0 and 1 depending on whether the local value is determined at an adhesive stripe or at an adhesive-free stripe, i.e. between two adhesive stripes. Fig. 8a also indicates two integration intervals Int 1 and Int 2, respectively, which are used to calculate the mean adhesive surface coverage of Fig.'s 8b and 8c, respectively.

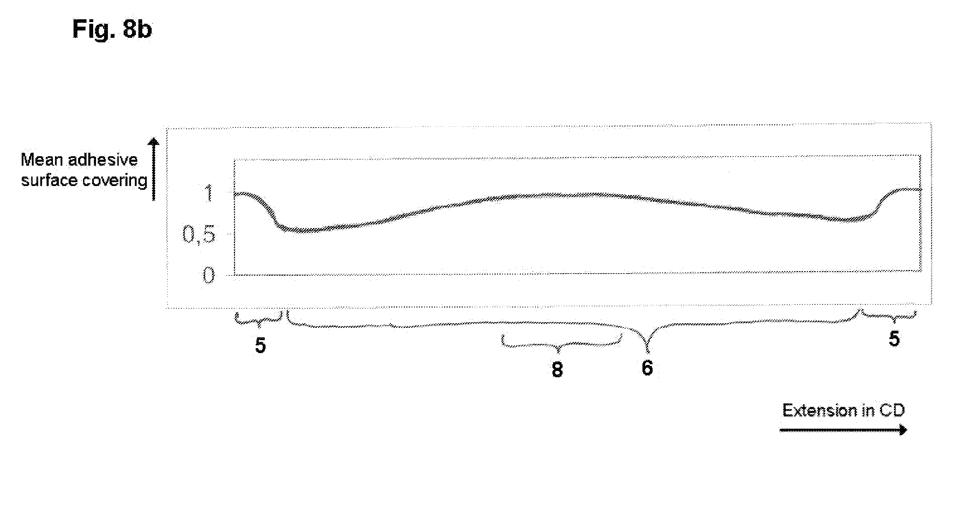

Figure 8b schematically shows the mean adhesive surface coverage calculated from the local adhesive surface coverage shown in Figure 8a using the interval Int 1. Since Int 1 is chosen as the sum of the width of the widest adhesive stripe 7 plus the width of the adhesive-free stripes 7a between the adhesive stripes 7, the mean adhesive surface coverage calculated in this manner has a broad maximum at the center of the intermediate region 6 close to but smaller than 1 and decreases towards the edges of the intermediate region to a value of about 0.5. In the edge regions 5 the mean adhesive surface coverage again increases to a value close to 1.

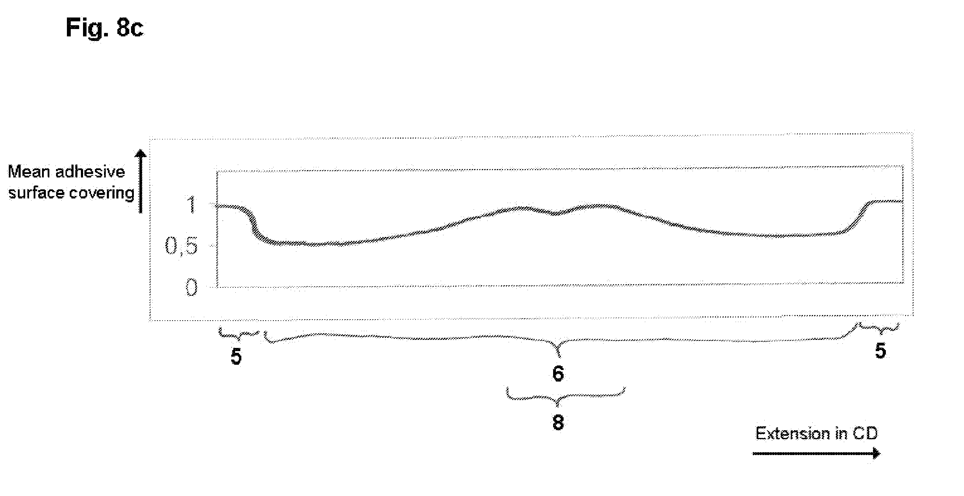

If a significantly smaller interval such as interval Int 2 of Figure 8a is chosen the resulting mean area coverage curve is less smooth as can be seen from Fig. 8c. Since the interval Int 2 is smaller than the sum of the width of the widest adhesive stripe 7 plus the constant width of the adhesive-freestripes 7a, the mean adhesive surface coverage has two local maxima in the center region 8 proximate the center line 9 as is schematically shown in Figure 8c. However, it is apparent to the skilled person that the occurrence of two maxima in the center region separated by a minimum is due to a less favorable choice of the integration interval Int 2. Choosing a more appropriate integration interval such as Int 1 used in Fig. 8b, provides a plot of the mean adhesive surface coverage versus the extension in CD that has one maximum in the center region 8.

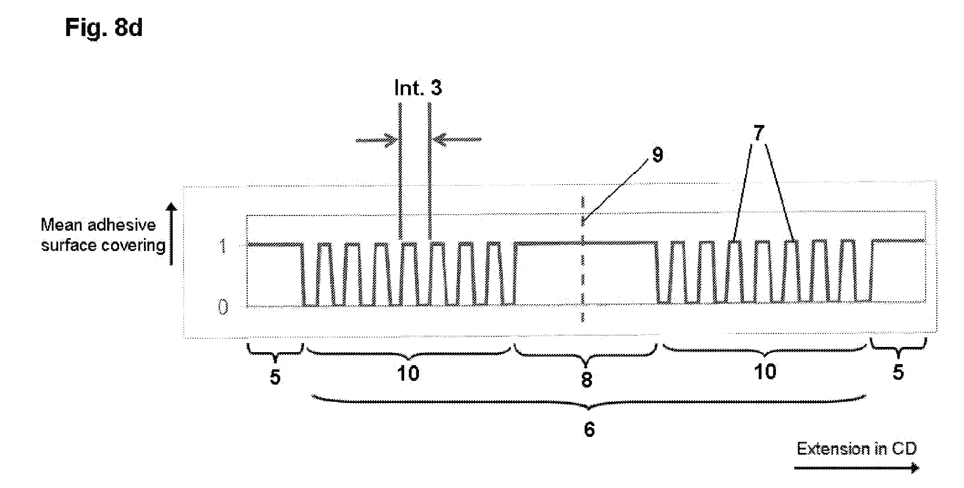

Figure 8d shows the local adhesive surface coverage in the intermediate region for the precursor of a composite laminate web shown in Figure 3a.

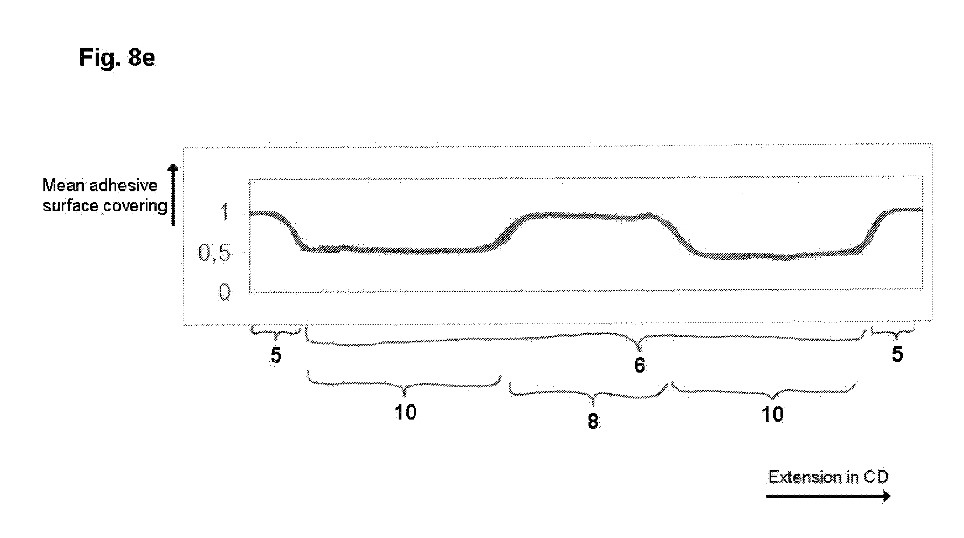

Figure 8e shows the mean adhesive surface coverage calculated from the local area coverage of Figure 8d using the integration interval Int 3 indicated in Fig. 8d which is defined by the width of an adhesive stripe 7 within the middle region plus the width of one adhesive-free stripe 7a in the middle region. The resulting mean area coverage shown in Figure 8e exhibits 3 maxima with a value of the mean adhesive surface area coverage of 1 in the two edge regions and in the center region which are separated by broad middle regions having a value of the mean adhesive surface coverage of about 0.5.

Fig.'s 9a and 9b are described in Example 2 below.

### List of reference signs

- 1: activatable precursor of the composite laminate web or the corresponding activated composite laminate web
- 2: elastic layer
- 3: first adhesive layer
- 3a: second adhesive layer
- 4: first non-woven web
- 4a: second non-woven web
- 5: edge region
- 6: intermediate region
- 7: adhesive stripe
- 7a: adhesive-free stripe
- 8: center region
- 8a: secondary center region
- 8': edge region obtained from a center region or secondary center region, respectively, upon cutting
- 9: center line
- 10: middle region
- 11: cut-out
- 11a: left panel
- 11b: right panel
- 12: fastening tabs/hook tabs
- 13: cut line

### Examples

### Materials used in the Examples

- Carded nonwoven web Sawabond 4179 which is commercially available from Sandler AG, Schwarzenbach, Germany. Sawabond 4179 is made from PP (polypropylene) staple fibers having a pre-processing fibre elongation at break of about 250-400 %. The carded nonwoven web is thermo-bonded in a calander apparatus fitted with 7-15 % bonding area at a temperature of about 145-155 °C
   Further properties of Sawabond 4179:
   - basis weight about 22 g/m²
   - fiber titer about 2,2 dtex
   - staple fiber length about 40mm
   - about 20-30 essentially homogeneously distributed bonding points/cm²
   - fiber orientation about 5-6 to 1((MD/CD)
- Activatable elastic film UKME 50 which is commercially available from 3M Co., St. Paul, U.S.A. UKME 50 is a three-layer film with two outer skin layers of homopolypropylene (PP) (melt flow index of 18 g/10 min) which is commercially available as 8069 Polypropylene from Total Petrochemicals, Feluy, Belgium, with a thickness of 3 µm each and an elastomeric core layer using styrene-isoprene-styrene (SIS) / polystyrene (PS) (70:30) polymers. The SIS used is a 100 % triblock styrene-isoprene-styrene which is supplied by Kraton Polymers, Pernis, The Netherlands, as Kraton D1114, Kraton 1160 SIS Rubber. The PS used has a melt flow rate of 13 cm³/10 min and is available from Nova Chemicals, Carrington, UK, as Nova 3700 Crystal Grade PS. The core to skin ratio is 8.2:1 as the elastic core layer was of 49 µm thickness and the non-elastic skin layers were 3 µm thick. Hotmelt adhesive HX20025-02 commercially available from Bostik Company Netherland B. V., Roosendaal, The Netherlands.
- Blue colorant solution used for visualizing the adhesive pattern in the precursor web and the activated web, respectively, is prepared by dissolving 1 g Ceresblau commercially available from Bayer AG, Leverkusen, in 1 liter spirit. The obtained colorant solution was filtered after one hour.

### Example 1

### a) Preparation of an activated elastic composite laminate web

Two separate carded nonwoven webs Sawabond 4179 described above each having a width of 320 mm in CD were provided. On one major side of each nonwoven web hotmelt adhesive HX20025-02 commercially available from Bostik Company Netherland B. V., Roosendaal, The Netherlands, was applied in a pattern to provide the following zones from the left edge of the web (designated as 0 mm) to the right edge of the web (designated as 320 mm):
- 0-20 mm: continuously, fully adhesive coated left edge region 5
- 20-60 mm: middle region 10 having adhesives stripes 7 each having a width of 1.2 mm separated by adhesive-free stripes 7a each having a width of 1 mm
- 60-100 mm: continuously, fully adhesive coated secondary centre region 8a
- 100-140 mm: middle region 10 having adhesives stripes 7 each having a width of 1.2 mm separated by adhesive-free stripes 7a each having a width of 1 mm
- 140-180 mm: continuously, fully adhesive coated center region 5
- 180-220 mm: middle region 10 having adhesives stripes 7 each having a width of 1.2 mm separated by adhesive-free stripes 7a having a width of 1 mm
- 220-260 mm: continuously, fully adhesive coated secondary centre region 8a
- 260-300 mm: middle region 10 having adhesives stripes 7 each having a width of 1.2 mm separated by adhesive-free stripes 7a each having a width of 1 mm
- 300-320 mm: continuously, fully adhesive coated left edge region 5

The adhesive pattern is hotmelt coated onto each of the nonwoven webs by using an appropriately designed shim inserted between the upper and lower half of the die, respectively. The shim was designed so that it was mirror-inverted to the above adhesive pattern, i.e. the shim was a grid having slits where adhesive stripes are required and bars to produce essentially adhesive-free stripes therebetween, respectively. The mean coating density across the web was 4.7 g/sqm.

Then, an activatable elastic film UKME 50 web having a width in CD of 320 mm was provided. The first nonwoven web and the UKME 50 web were fed into a nip so that the surface of thenonwoven web layer bearing the hotmelt adhesive layerswas facing the skin layer on one surface of the UKME 50 web. The nip was formed by a steel roll and another rubber roll without any additional temperature or cooling and the nonwoven webs and the activatable elastic laminate web were laminated under zero strain conditions. Subsequently, the second non-woven layer was applied to the opposite side of the UKME web.

The resulting precursor of the composite laminate web was activated in a diverging disk stretching apparatus as described above by a stretch ratio of 180 % (to a total elongation of 280 %) in CD using the following stretching program:
Full stretch takes 0.72sec @100m/min and 0.36sec @ 200m/min (based on 1200mm guided web in the stretcher from zero stretch (160mm) up to maximum stretch (448mm)
448mm per 0.72sec -> 37333,33 mm/min stretch rate @100m/min line speed
448mm per 0.36sec -> 74666,67mm/min stretch rate @ 200m/min line speed

The cross-section of the precursor web and the activated web obtained from it by stretching corresponds to the cross-section shown in Fig. 3b.

### b) Recording of hysteresis curves

A first piece of the activated composite laminate web referred to as SAMPLE 1 having an extension of 40 mm in MD and 80 mm in CD was cut so that it comprised the middle area 10 of the activated composite laminate web which was neighbored in the direction to the left by a 20 mm wide edge region and in the direction to the right by a 20 mm wide section of the center region 8 and the secondary center region 8a, respectively. The middle region comprised adhesives stripes each having a width of 1.2 mm separated by adhesive-free stripes having a width of 1 mm.

A second piece of the activated composite laminate web referred to as SAMPLE 2 having an extension of 40 mm in MD and 80 mm in CD was cut symmetrically around the center region 8 so that the middle of SAMPLE 2 in CD corresponded to the center region 8 which was neighbored in each direction to the left and right by a 20 mm wide area of the middle region 10. Thus, the configuration of SAMPLE 2 in CD, from left to right, was:
20 mm of the middle region 10; 40 mm of the center region 10; 20 mm of the middle region

Hysteresis curves were then recorded for SAMPLES 1 and 2, respectively, as follows. The respective SAMPLE was mounted in a tensile testing machine (Zwick™ Model Z005 available from Zwick) so that it could be extended in CD. Line-contact jaws were used to minimize slip and breakage in the jaws.

In the measurements described below the upper and lower jaws of the tensile testing machine were 60 mm apart so that the respective sample was exceeding the jaw lines on both ends of its extension in CD by 10 mm. The instrument cross head speed of the tensile testing machine was set in each case at a rate of 500 mm/min, and the extension was progressed until a force of 10 N was reached. At the end point the jaw moved backwards without a holding time (1^{st} run), and then a 2^{nd} run was recorded.

The hysteresis curve recorded for SAMPLE 2 is shown in Fig. 7a. The hysteresis curve recorded for SAMPLE 1 is shown in Fig. 7b. Fig. 7c shows a comparison of the hysteresis curves for SAMPLES 1 and 2, respectively. It can be seen that SAMPLE 2 is less elastic than SAMPLE 1 because the maximum extension reached for a force of 10 N is lower for SAMPLE 2 in comparison with SAMPLE 1. This different elasticity behaviour is due to the different patterns of the adhesive layer present in SAMPLES 1 and 2, respectively.

### c) Shear strength measurements

A third piece of an activated elastic composite laminate web referred to below as SAMPLE 3 having an extension of 40 mm in MD and 80 mm in CD was obtained by cutting the web obtained in Example 1 a) above along a line parallel to and 80 mm apart in CD from the outer edge line of the edge region so that the resulting SAMPLE 3 exhibited, from left to right in CD, a fully adhesive coated left edge area 20 mm wide corresponding to the edge region 5 of the composite laminate web; an adhesive-stripe coated area 40 mm wide corresponding to the middle area of the composite laminate web; and a fully adhesive coated right edge area 20 mm wide corresponding to half of the seciondary center region 8a of the composite laminate web (edge region 8'). A mechanical closure tape tab designated as CHL-1732 having a width of 30 mm in MD is provided which is commercially available from 3M Deutschland GmbH, Neuss, Germany. The tape tab is adhesive-bonded to the fully-adhesive coated left edge area and right edge area, respectively, of SAMPLE 3 whereby the tab was attached in each case in a Y-bond fashion. The Y-bond extended on one surface of the composite web in a width of 15 mm in CD and on the other surface in a width of 17 mm in CD. The bonding area between the tab and the corresponding edge of the composite laminate web was thus in each case A = (15 mm x 30 mm) + (17 mm x 30 mm) = 9,6 cm². The bonding area was in each case rolled over twice (forward and backward) with a weight of 5 kg by hand.

SAMPLE 3 was then mounted in a tensile testing machine (Zwick™ Model Z005 available from Zwick) so that the tape tab attached to the left side of SAMPLE 3 (corresponding to the edge region 5 of the composite laminate web) was mounted in the left jaws of tensile testing machine. The right jaws were arranged in parallel in a distance of about 60 mm so that such jaws clamped the full width of the composite laminate web. The jaws were then separated with a speed of 500 mm/min until the tape tab was ripped off the composite laminate web. This test measured the shear strength between the tape tap and the edge region 5 of the composite laminate web. The force measured was about 46 N/9.6 sqcm (i.e. per tab).

In another test, the tape tab attached to the right side of SAMPLE 3 (corresponding to the center region 8 of the composite laminate web) is mounted in the jaws on one side of the tensile testing machine. The oppowsite jaws were arranged in parallel in a distance of about 60 mm so that such jaws clamped the full width of the composite laminate web. The jaws were then separated with a speed of 500 mm/min until the tape tab was ripped off the composite laminate web. This test measured the shear strength between the tape tap and the secondary center region 8a of the composite laminate web. The force measured was about 46 N/9.6 sqcm (i.e. per tab).

### Example 2

### a) Preparation of a precursor of a composite laminate web and of an activated composite laminate web, respectively

The composite laminate web was prepared as described in Example 1 except that the width of the adhesive stripes 7 was 1.0 mm and the width of the adhesive-free stripes was 2.0 mm.

### b) Measurement of adhesive pattern in the precursor web and the activated web, respectively

Furthermore, the precursor web and the composite laminate web, respectively, were coloured with the blue colorant solution as described above for visualizing the adhesive pattern. The blue colorant dissolves to a different extent in the elastic layer, the fiber web and the adhesive, respectively.

A piece having an extension in CD of about 30 mm was cut from the middle region of the precursor of the composite laminate web. This piece is referred to below as SAMPLE 4. Likewise, a piece having an extension in CD of about 30 mm was cut from the middle region of the activated composite laminate web. This piece is referred to below as SAMPLE 5.

Then one of the non-woven webs was carefully removed from each of SAMPLE 4 and 5 thereby exposing the adhesive pattern underneath.

Then, a micro-photo was taken from the side from which the non-woven layer had been removed using a Leica MZ 12 microscope, commercially available from company Leica Microsystems, Wetzlar/GERMANY. The widths of the adhesive stripes 7 and of the adhesive-free stripes 7a were measured. The microphotographs taken for the precursor web and the activated web, respectively, are reproduced in Fig.'s 9a and 9b, respectively.

The mean width of the adhesive-free stripes 7a of SAMPLE 4 was 2,0531 (with a standard deviation of 0,1022) mm. The deviation of the width of the adhesive-free stripes originally applied by the shim to web (2 mm) is thus low.

The mean width of adhesive strips 7 of SAMPLE 4 was 0,9886 (with a standard deviation of 0,0773) mm. The deviation of the width of the adhesive stripes originally applied by the shim to web (1 mm) is thus low.

For the activated SAMPLE 5, the mean width of the adhesive-free stripes was measured as 2,2073 mm (with a standard deviation of 0,1701 mm). The deviation of the width of the adhesive stripes originally applied by the shim to web (2 mm) is thus low.

For the activated SAMPLE 5, the median width of the adhesive stripes was measured as 0,9240 mm (with a standard deviation of 0,0445 mm). The deviation of the width of the adhesive stripes originally applied by the shim to web (1 mm) is thus low.

### Comparative Example 1

### a) Preparation of an activated elastic composite laminate web

A composite laminate web was prepared as described in Example 1 except that the pattern of the adhesive was, from the left edge of the web (designated as 0 mm) to the right edge of the web (designated as 320 mm), as follows:
0-20 mm: continuously, fully adhesive coated left edge region 5
20-140 mm: middle region 10 having adhesives stripes each having a width of 1.2 mm separated by adhesive-free stripes having a width of 1 mm
140-180 mm: continuously, fully adhesive coated centre region 8
180-300 mm: middle region 10 having adhesives stripes each having a width of 1.2 mm separated by adhesive-free stripes having a width of 1 mm
300-320 mm: continuously, fully adhesive coated left edge region 5

### b) Shear strength measurements

A piece of an activated elastic composite laminate web referred to below as SAMPLE 6 having an extension of 40 mm in MD and 80 mm in CD was obtained by cutting the web along the centre line 9 and along a parallel line 80 mm apart in CD so that the resulting SAMPLE 6 exhibited, from left to right in CD, a fully adhesive coated left edge area 8' 20 mm wide corresponding to half of the center region 8 of the composite laminate web and an adhesive-stripe coated area 60 mm wide corresponding to the middle area of the composite laminate web. A mechanical closure tape tab designated as CHL-1732 having a width of 30 mm in MD is provided which is commercially available from 3M Deutschland GmbH, Neuss, Germany. The tape tab is adhesive-bonded to the right edge of SAMPLE 6, i.e. to the middle region comprising adhesive-stripes, whereby the tab was attached in a Y-bond fashion as described above in Example 2. The bonding area between the tab and the middle region of the composite laminate web was thus A = (15 mm x 30 mm) + (17 mm x 30 mm) = 9,6 cm². The bonding area was in each case rolled over twice (forward and backward) with a weight of 5 kg by hand.

SAMPLE 6 was then mounted in a tensile testing machine (Zwick™ Model Z005 available from Zwick) so that the tape tab attached to the right side of SAMPLE 6 (corresponding to the middle region 10 of the composite laminate web) was mounted in jaws of the tensile testing machine. The other opposite jaws were arranged in parallel in a distance of about 60 mm so that such jaws clamped the full width of the composite laminate web. The jaws were then separated with a speed of 500 mm/min until the tape tab was ripped off the composite laminate web. This test measured the shear strength between the tape tap and the middle region 10 of the composite laminate web. The force measured was about 36 N/9.6 sqcm (i.e. per tab).

## Claims

1. Activatable precursor of an elastic composite laminate web having a machine direction and a cross direction comprising an elastic film, a first non-woven web and a first layer of adhesive therebetween, said layer of adhesive comprising in CD two edge regions and an intermediate region between the two edge regions extending in MD, said layer of adhesive exhibiting an adhesive pattern so that the mean adhesive surface coverage measured in the cross direction comprises at least one maximum in each of the edge regions and at least one maximum proximate the center of the intermediate region and/or proximate to the center line of the web, respectively.

2. Activatable precursor of an elastic composite laminate web according to claim 1 wherein the edge regions are fully coated with adhesive.

3. Precursor according to any of the preceding claims wherein the width of said edge regions is between 10 and 30 mm, preferably between 15 and 25 mm.

4. Activatable precursor of an elastic composite laminate web according to any of the previous claims wherein said maxima of the mean adhesive surface coverage are arranged essentially equally spaced from each other.

5. Activatable precursor of an elastic composite laminate web according to any of the previous claims comprising at least two further maxima of the mean adhesive surface coverage which are arranged in the middle regions between the center region and the edge regions, respectively.

6. Precursor according to any of the previous claims wherein the intermediate region comprises stripes of adhesives oriented in the machine direction which are separated by stripes essentially free of adhesives.

7. Precursor according to claim 6 wherein said adhesive stripes comprise a central adhesive stripe proximate the center of said intermediate region whose width in CD is between 20 and 60 mm, preferably between 30 and 50 mm.

8. Precursor according to claim 7 wherein the adhesive stripes between the center stripe and the edge regions are of constant width.

9. Precursor according to claim 8 wherein the width of said stripes varies between 0.5 and 5 mm, preferably between 0.8 and 2 mm.

10. Precursor according to any of claims 6 - 9 wherein width the stripes essentially free of adhesive is between 0.5 and 5 mm, preferably between 0.8 and 2 mm.

11. Precursor according to any one of the preceding claims wherein the elastic film is an activatable elastic film.

12. Precursor according to any of the preceding claims wherein the first and/or second non-woven webs comprise carded non-wovens.

13. Precursor according to any of the preceding claims wherein the elastic film continuously extends over the width of the precursor and/or the activated laminate in CD.

14. Elastic composite laminate web obtainable by stretching in the CD the precursor according to any of claims 1-13.

15. Method of manufacturing a precursor of a composite laminate web having a machine direction and a cross direction, in particular a precursor of a composite laminate web according to any one of claims 1 - 13, said method comprising the following steps:
a) providing a non-woven web having first and second surfaces;
b) providing a layer of adhesive on a surface of the non-woven web, said layer of adhesive comprising in CD two edge regions and an intermediate region between the two edge regions extending in MD, said layer of adhesive exhibiting an adhesive pattern so that the mean adhesive surface coverage measured in the cross direction comprises at least one maximum in each of the edge regions and at least one maximum proximate the center of the intermediate region and/or the center line of the web, respectively,
c) providing an elastic film having first and second surfaces, and
d) attaching the surface of the non-woven web bearing said layer of adhesive, to a surface the elastic film.
